(19) Europäisches Patentamt
European Patent Office
Office européen des brevets

(11) **EP 3 205 263 B1**

(12) **EUROPEAN PATENT SPECIFICATION**

(45) Date of publication and mention
of the grant of the patent:
**19.06.2019 Bulletin 2019/25**

(51) Int Cl.:
***A61B 5/021*** *(2006.01)*

(21) Application number: **16002744.7**

(22) Date of filing: **02.04.2013**

(54) **BLOOD PRESSURE ESTIMATION USING A HAND-HELD DEVICE**

BLUTDRUCKEINSCHÄTZUNG UNTER VERWENDUNG EINER TRAGBAREN VORRICHTUNG

ESTIMATION DE PRESSION SANGUINE À L'AIDE D'UN DISPOSITIF À MAIN

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **29.03.2012 US 201213433608**
**03.01.2013 US 201313733235**

(43) Date of publication of application:
**16.08.2017 Bulletin 2017/33**

(62) Document number(s) of the earlier application(s) in
accordance with Art. 76 EPC:
**13161986.8 / 2 644 089**

(73) Proprietor: **LifeWatch Technologies Ltd.**
**76100 Rehovot (IL)**

(72) Inventors:
• **Yakirevich, Sergey**
  **58459 Holon (IL)**
• **Tal, Yair**
  **45858 Matan (IL)**
• **Tal, Benny**
  **78374 Ashkelon (IL)**
• **Pressman, Assaf**
  **85338 Lehavim (IL)**

(74) Representative: **Graf von Stosch, Andreas et al**
**Graf von Stosch**
**Patentanwaltsgesellschaft mbH**
**Prinzregentenstraße 22**
**80538 München (DE)**

(56) References cited:
**US-A1- 2010 222 652**

• **HEIKO GESCHE ET AL: "Continuous blood pressure measurement by using the pulse transit time: comparison to a cuff-based method", EUROPEAN JOURNAL OF APPLIED PHYSIOLOGY, SPRINGER, BERLIN, DE, vol. 112, no. 1, 10 May 2011 (2011-05-10), pages 309-315, XP019999107, ISSN: 1439-6327, DOI: 10.1007/S00421-011-1983-3**
• **Qiao Zhang: "CUFF-FREE BLOOD PRESSURE ESTIMATION USING SIGNAL PROCESSING TECHNIQUES", A Thesis Submitted to the College of Graduate Studies and Research in Partial Ful Ilment of the Requirements for the degree of Master of Science in the Division of Biomedical Engineering University of Saskatchewan Saskatoon, 1 August 2010 (2010-08-01), XP055385151, Retrieved from the Internet: URL:https://pdfs.semanticscholar.org/1f63/ 18e26a11eed44e884f480338aa82f6918bbe.pdf [retrieved on 2017-06-26]**

EP 3 205 263 B1

Description

## BACKGROUND OF THE INVENTION

Blood pressure measurements are cumbersome or in accurate. Cuff based solutions require either expensive or inaccurate measurement equipment and are cumbersome. There is a growing need to provide a systems and methods for blood pressure measurements that are inexpensive and easy to implement.

## SUMMARY OF THE INVENTION

According to the invention there are provided devices, computer readable media and methods for blood pressure measurements according to claims 12, 13 and 1, respectively.

## PRIOR ART

US 2010/222652 A1 teaches a method for providing a blood pressure indicator of a person, wherein the method comprises obtaining multiple first detection signals from a non-invasive optical phthysmography sensor and obtaining multiple second detection signals from a non-invasive electrocardiography sensor. The multiple first and second detection signals are processed and a blood pressure indicator is calculated.

## BRIEF DESCRIPTION OF THE DRAWINGS

The subject matter regarded as the invention is particularly pointed out and distinctly claimed in the concluding portion of the specification. The invention, however, both as to organization and method of operation, together with objects, features, and advantages thereof may best be understood by reference to the following detailed description when read with the accompanying drawings in which:

Figs. 1A-1C illustrate hand-held devices according to various embodiments of the invention;
Figs. 2A-2B illustrate hand-held devices according to various embodiments of the invention;
Figs. 3A-3B illustrate hand-held devices according to various embodiments of the invention;
Fig. 3C illustrates a portion of the hand-held device of any of figures 1A-1C, 2A-2B and 3A-3B, according to an embodiment of the invention;
Figs. 4A-4C illustrate a hybrid sensor according to various embodiments of the invention;
Figs. 5A-5C illustrate a hybrid sensor according to various embodiments of the invention;
Fig. 6 illustrates a method according to an embodiment of the invention; and
Fig. 7 illustrates a method according to an embodiment of the invention;

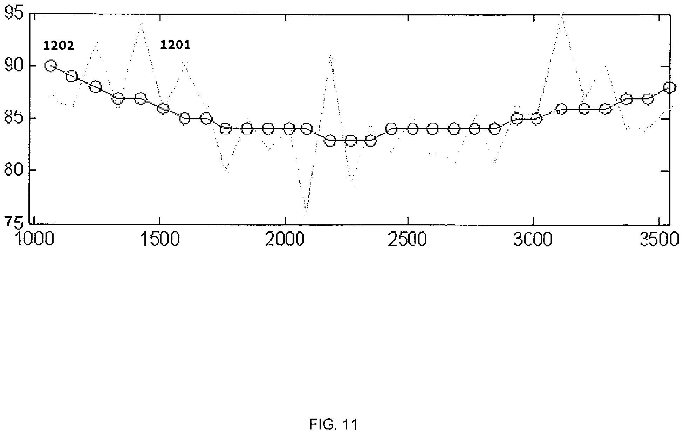

Figs. 8-12 illustrate various signals according to various embodiments of the invention;
Figs. 13-14 illustrate various signals according to various embodiments of the invention; and
Figures 15-17 illustrate various methods according to various embodiments of the invention.

It will be appreciated that for simplicity and clarity of illustration, elements shown in the figures have not necessarily been drawn to scale. For example, the dimensions of some of the elements may be exaggerated relative to other elements for clarity. Further, where considered appropriate, reference numerals may be repeated among the figures to indicate corresponding or analogous elements.

## DETAILED DESCRIPTION OF THE DRAWINGS

The subject matter regarded as the invention is particularly pointed out and distinctly claimed in the concluding portion of the specification. The invention, however, both as to organization and method of operation, together with objects, features, and advantages thereof, may best be understood by reference to the following detailed description when read with the accompanying drawings.

In the following detailed description, numerous specific details are set forth in order to provide a thorough understanding of the invention. However, it will be understood by those skilled in the art that the present invention may be practiced without these specific details. In other instances, well-known methods, procedures, and components have not been described in detail so as not to obscure the present invention.

The following abbreviations and terms are used in this specification:

| HR | Heart Rate |
| --- | --- |
| BE | Backend of a hand-held device. It can be a server which among other tasks runs the algorithm on data which was sent from the hand-held device. |
| QRS | A waveform presented in an ECG during ventricular depolarization |
| RR interval | Distance (time) between sequential QRS complexes - two consecutive R waves |
| PTT | Pulse Transient Time. Time between the occurrence of the QRS complex and the corresponding PPG pulse. |
| Tachycardia | A rapid heart rate, especially one above 100 beats per minute in an adult |

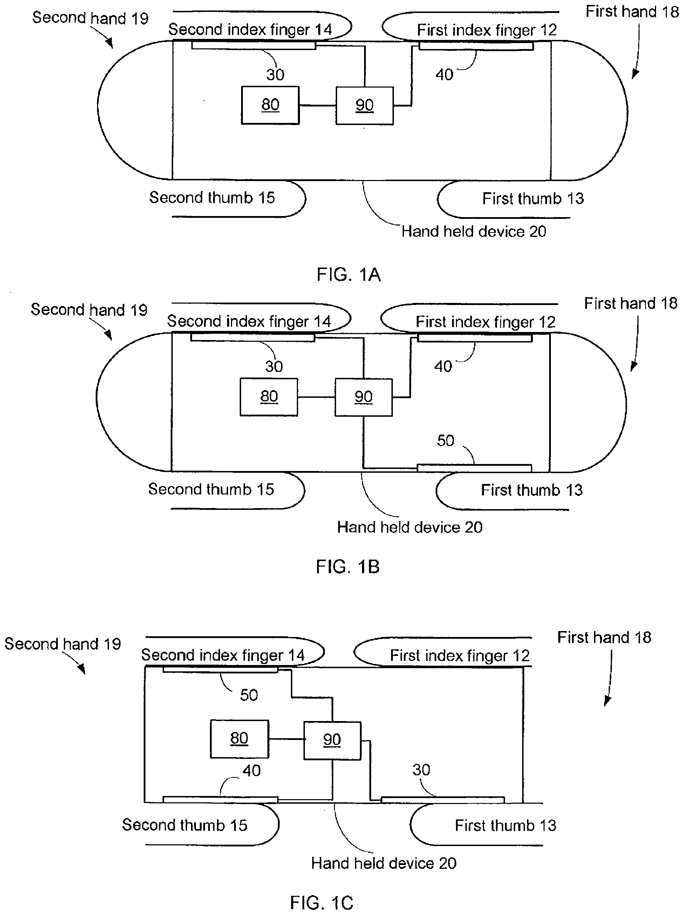

There is provided a compact, cheap and resilient hand-held device that has health monitoring capabilities. The hand-held device can include one or more sensors that are integrated with a smart phone, a media player, a game console, a communication device, a mobile phone, a palm computer and the like.
The device is hand-held in the sense that it can be held by one or two hands of a user. The user can hold the hand-held device with one hand, the device can be attached to a user or to another user accessory but the user can be requested to hold the hand-held device by one or two hands when performing at least one medical examination.
The shape of the hand-held device can be rectangular (as illustrated in figures 1A-1B, 2A-2B and 3A-3B) but can have other shapes such as an oval shape, elliptical shape, a polygon shape and the like.
The hand-held device can include multiple medical sensors that may include electrodes, optical elements, infrared elements, chemical sensors and the like. One or more of these sensors can be a hybrid sensor that can include different types of sensing elements such as electrodes and light sensing elements.
Figures 1A, 1B, 1C, 2A, 2B, 3A and 3C illustrate various examples of hand-held devices 20 that are contacted by users. The following table illustrates the mapping between fingers and sensors (30, 40, 40', 50, 50' 50") that should be contacted by the user, according to various embodiments of the invention.

| figure | First hand 18 | | Second hand 19 | |
| --- | --- | --- | --- | --- |
| | 1st index finger 12 | 1st thumb 13 | 2nd index finger 14 | 2nd thumb 15 |
| 1A | 40 | | 30 | |
| 1B | 40 | 50 | 30 | |
| 1C | | 30 | 50 | 40 |
| 2A | 40 | 50 | 30 | 60 |
| 2B | 40 | 30 | 50, 50' | |
| 3A | 40, 40' | 50, 50', 50" | 30 | 60 |
| 3B | | 30 | 50, 50' | 40 |

Figure 1A illustrates a hand-held device 20 that is being held by two hands (18 and 19) of a user. The hand-held device 20 may include: (a) a first sensor 40 that is positioned such as to be contacted by a first hand 18 of a user when the user holds the hand-held device 20; (b) a second sensor 30 that is positioned such as to be contacted by a second hand 19 of the user when the user holds the hand-held device; and (c) a health monitoring module 90 arranged to process detections signals from the electrodes and from the light detector such as to provide processed signals that are indicative of a state of the user. The health monitoring module 90 can perform the entire processing, can perform a partial processing and then send (or assist in sending) the partially processed signals to another entity (such as the main processor of the hand held device, a remote processing entity, a medical hub, a hospital etc) to be further processed. The health monitoring module 90 can be dedicated for medical processing or can be also allocated to other tasks. The health monitoring module 90 can be a general purpose processor or a digital signals processor, it can control the functionality of the hand-held device 20.
Either one of the first sensor 40 and the second sensor 30 can be placed on (or embedded with) an edge or a surface of the hand-held device 20 so that once the user touches that edge or surface, the user may touch the first sensor 40.
Figures 1A and 1B illustrate the first sensor 40 and the second sensor 30 as belonging to a top side of the hand-held device 20 while figure 1C illustrates the first sensor 40 and the second sensor 30 as belonging to a bottom side of the hand-held device 20.

The first and second sensors 40 and 30 can be located at the same side of the hand-held device 20, can be positioned at different sides and even opposite sides of the hand-held device 20. For example, first sensor 40 can be positioned at a top side of the hand-held device 20 while the second sensor 30 can be positioned at a bottom side, a sidewall, a back side or even at the front panel of the hand-held device 20.

Figure 1A also illustrates the hand-held device 20 as including a man machine interface (MMI) element 80. This MMI element 80 can be a screen, a keyboard, a microphone, a loudspeaker, a touch screen and the like. This MMI element 80 can be much bigger than is being illustrated in Figure 1A. It can span across the entire (or almost entire) hand held device 20. Yet according to another embodiment of the invention one or more sensor is connected to the application processor of the hand held device.

The MMI element 80 can provide to the user instructions to be followed during the medical test. For example, the MMI element 80 can request a user to contact one or more sensors, to limit the movement of the user, to change position or try to clean an electrode if it is detected that a certain electrode does not receive goon enough (too noisy or too weak) signals, and the like. The MMI element 80 can display or otherwise make the user aware of the outcome of the medical evaluation.

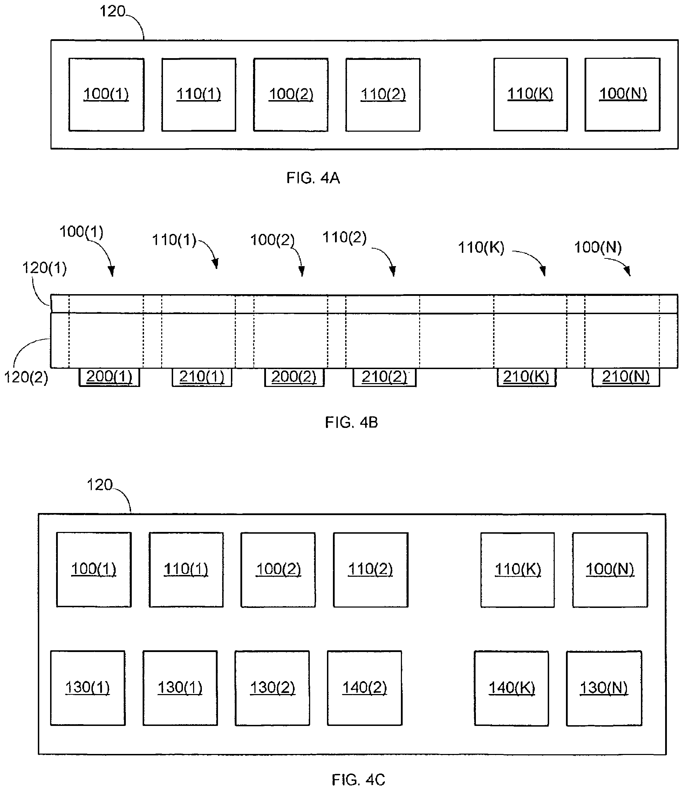

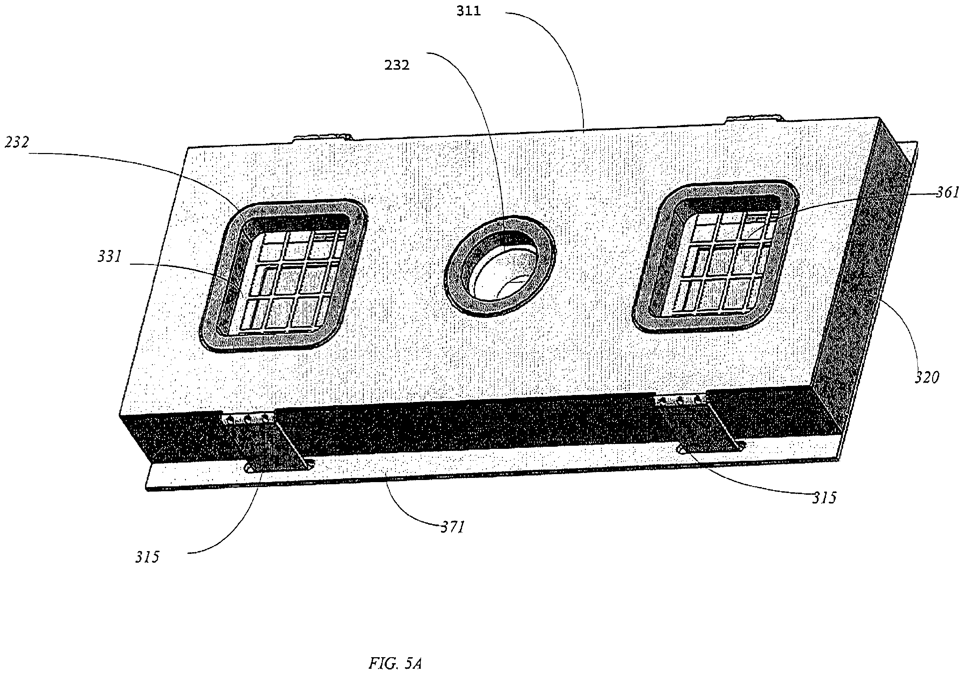

At least one sensor out of the first sensor 40 and the second sensor 30 can be a hybrid sensor that may include an electrode, an illumination element and a light detector. Non-limiting examples of a hybrid sensor (denoted 70) are shown in figures 4A-4C and 5A-5C.

According to an embodiment of the invention the hand-held device 20 can include more than two sensors. It can include for example, a third sensor such as third sensor 50 of figures 1B and 1C, 2A, 2b, 3A and 3B.

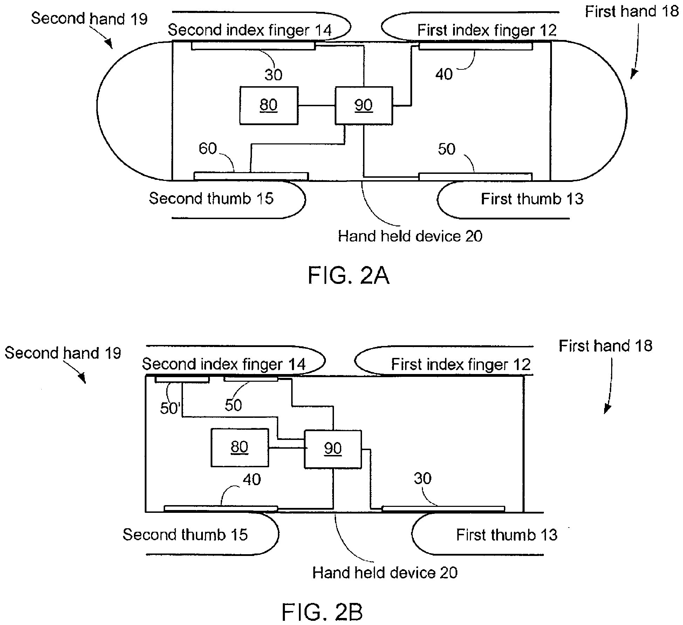

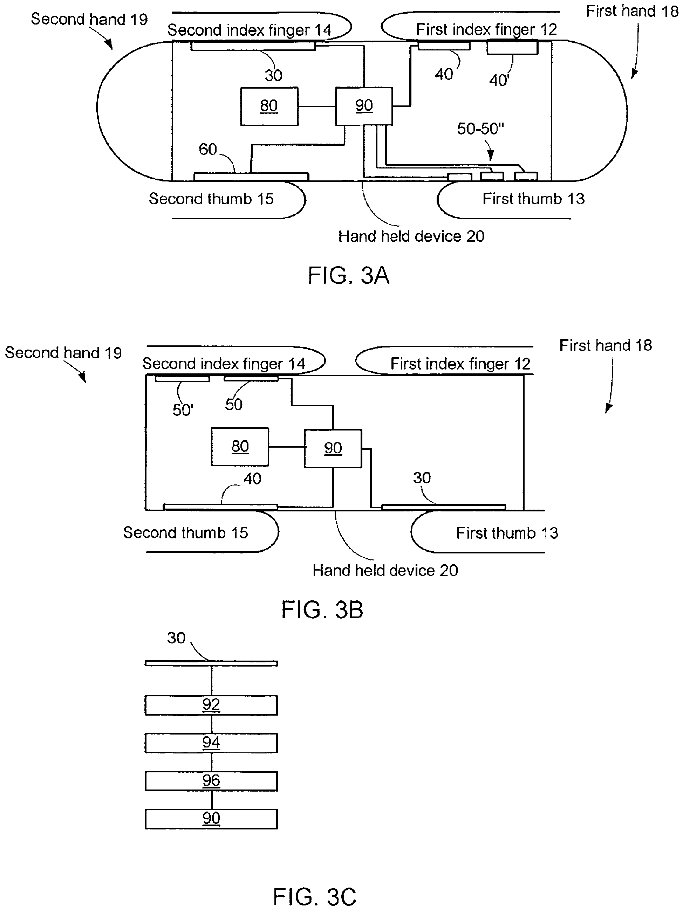

Yet for another example, the hand-held device 20 can include a fourth sensor, such as fourth sensor 60 of figures 2A, 3A and 50' of figure 3B.

Yet for a further example, the hand-held device 20 can include a fifth sensor, such as fifth sensor 40' of figure 3A, can include a sixth sensor such as sixth sensor 50' of figure 3A and can include a seventh sensor such as seventh sensor 50" of figure 3A.

The number of sensors of the hand-held device can exceed seven.

The sensors can be positioned such that each sensor is touched by a different finger of the user (as illustrated in figures 1A, 1B, 1C, 2A, 2B) although multiple sensors can be positioned such as to be touched by the same finger of the user (as illustrated in figures 3A and 3B). The number of sensors that can be touched by the same finger can be two, three or more.

Fig. 3C illustrates a portion of the hand-held device of any of figures 1A-1C, 2A-2B and 3A-3B, according to an embodiment of the invention. Figure 3A illustrates that a sensor (such as second sensor 30) is coupled to the health processing module 90 via analog circuits such as amplifier 92, mixed signal circuits such as analog to digital converter (ADC) 94 and memory unit 96. Electrical detection signals from an electrode of the second sensor 30 are amplified to amplifier 92 to provide amplified detection signals. The amplified detection signals can converted to digital detection signals that can be stored in memory unit 96 and/or processed by health monitoring module 90.

The following figures illustrate a hybrid sensor. It is noted that this is merely a non-limiting example and that other sensors can be used.

Figures 4A and 4B are top and side views of a hybrid sensor 70 according to an embodiment of the invention.

The hybrid sensor 70 includes an electrode 120 that has apertures - light illumination apertures 110(1)- 1 10(K) and light collection apertures 100(1)-100(N). The user, or more specifically a finger of the user that touches the electrode (or is positioned above these apertures) is illuminated by light generated by illumination elements 210(1)- 210(K) and directed through the light illumination apertures 110(1)- 110(K). Light (scattered and/or reflected) from the finger passes through the light collection apertures 100(1)- 100(N) and is detected by light detectors 200(1)- 200(N). N and K are positive integers. N may differ from K but N may be equal K.

The electrode 120 is illustrated as including a conductive portion 120(1) that is supported by another portion 120(2).

While figures 4A and 4C illustrate a linear array of illumination elements and light detectors it is noted that the light detectors and light detectors can be arranged in other manners- for example, as a rectangular array - as illustrated by the two row array of figure 4A.

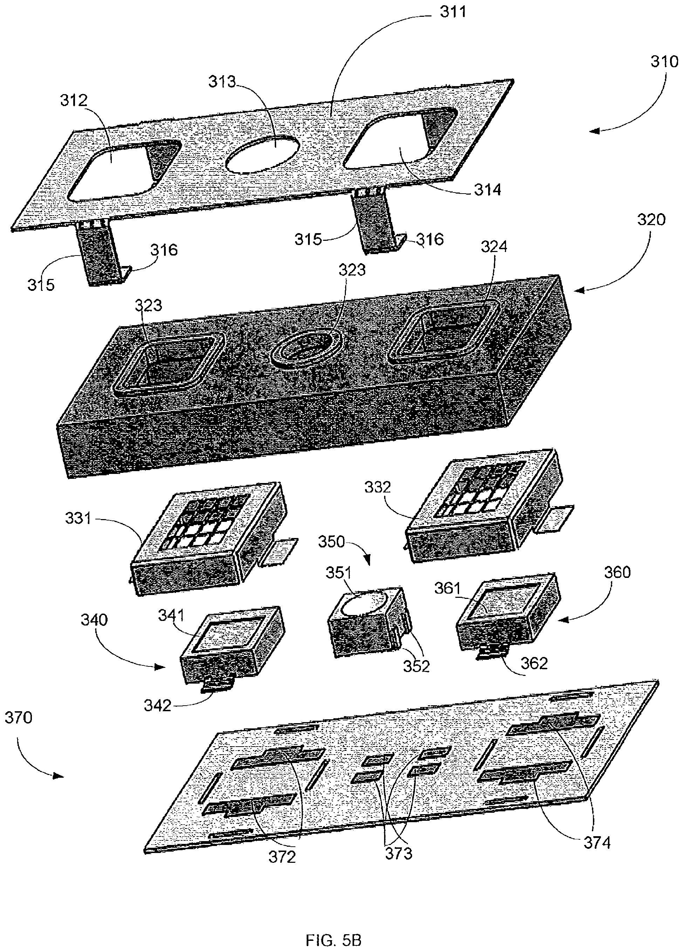

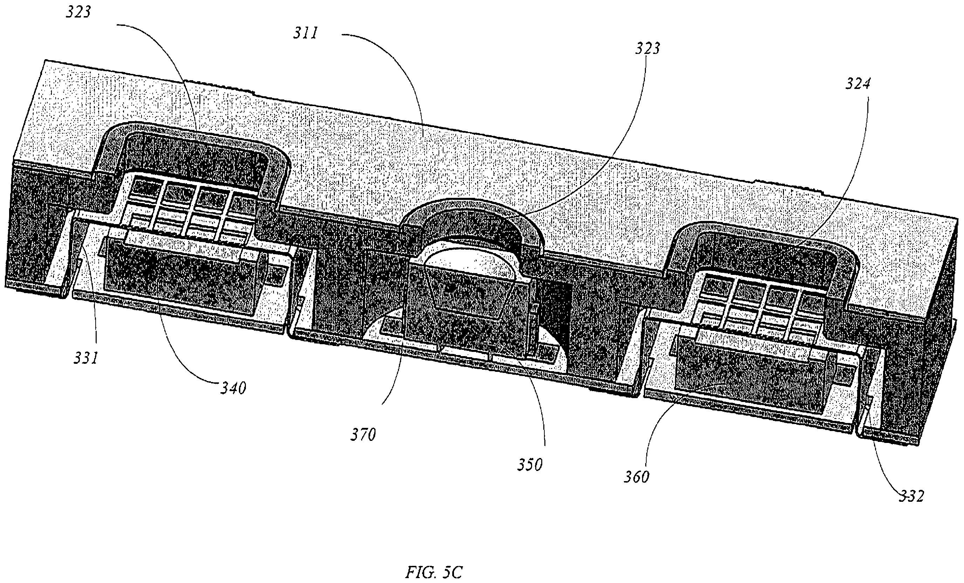

It is noted that the illumination elements and the light detectors can be arranged in an interleaved manner (as illustrated in figures 4A, 4B, 5A, 5B, and 5C) but can be arranged in other manners.

It is noted that unwanted artifacts and signal noises can be reduced by either one of using electrodes with low impedance, shielding power and signal lines and raising the input impedance of the amplifier.

Light from an illumination element can be collected by one or more light detectors. Figures 5A-5C illustrate a pair of light detectors per a single illumination element but the ratio can differ from 1:2. If there are more than one illumination elements then the number of light detectors associated with a single illumination element can differ from one illumination element to the other or can be equal to each other.

Figures 5A-5C provide a top view, an exploded view and a cross sectional view of a hybrid sensor 70 according to an embodiment of the invention.

The hybrid sensor 70 includes: (a) a conductive portion 310 of an electrode, (b) an additional portion 320 of the electrode, (c) protective shields 331 and 332, (d) illumination element 350, (e) light detectors 340 and 360, and (f) electrical circuit 370. The electrical circuit 370 can be a rigid or flexible electrical board that provides electrical connectivity (for power supply, control signals and communications) to the illumination element 350 and to light detectors 340 and 360. The electrical circuit 370 can be connected to a power supply source and to the health monitoring processor.

The conductive portion of the electrode 310 is positioned above other parts of the hybrid sensor 70. It has an upper surface 311 that defines a light illumination aperture 313 that is positioned between two light collection apertures 312 and 314. The upper surface 311 is connected to four supporting legs, each supporting leg is conductive and include a vertical plate 315 and a horizontal plate 316. The horizontal plate 316 can be connected to the board 371 of the electrical circuit 370. The electrical circuit 370 can have slits in which each leg can be inserted to that the horizontal plate 316 can be positioned below the board 317 and can be used for assisting in fastening the elements of the hybrid sensor 70 to each other.

The additional portion 320 of the electrode can provide mechanical support to the conductive portion 310 and can defined spaces (322, 323 and 324) that are positioned below apertures 312, 313 and 314 and allow light to be directed towards the user (through space 323) and be collected (via spaces 322 and 324).

The additional portion can be made of non-conductive material.

Protective shields 331 and 332, and light detectors 340 and 360 can be placed within spaces 322 and 324 while illumination element 350 can be placed within space 323. Each one of light detectors 340 and 360 and illumination element 350 can conductors (such as 342, 352 and 362) to provide electrical connectivity with conductors (372, 373 and 374) of the board 371.

The hand-held device 20 can activate one sensor or multiple sensors and can correlate or otherwise use detections signals from one sensor to evaluate detection signals from another sensor. For example, the electrode 310 can provide signals that are characterized by a low signal to noise ratio and thus various waveforms such as the QRS complex can be hard to detect. The light detector 350 can sense light that is indicative of a movement of the blood vessels of the user that corresponds to the QRS complex and this detection can be used for defining a time window in which to search for the QRS complex at the signals of the electrode. The time window is time shifted from the appearance of the QRS complex at the light detector signal due to a known delay between the generation of the RQS complex pulse and appearance of a movement that reflects the blood wave at the user's finger.

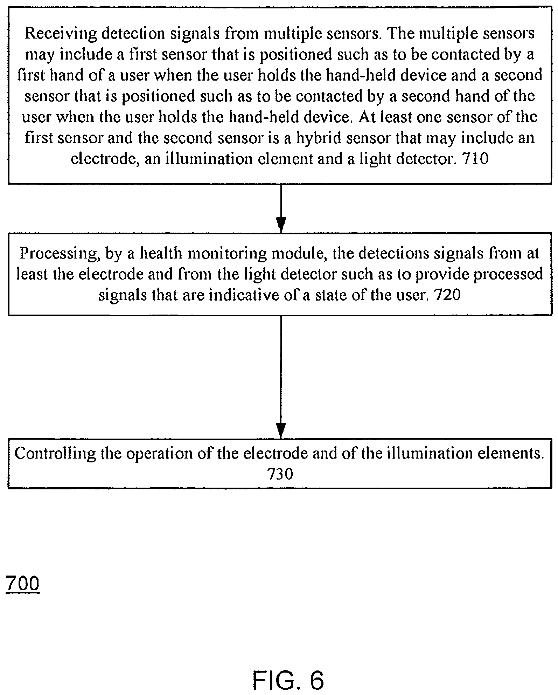

Figure 6 is a flow chart of a method 700.

Method 700 for monitoring a state of a user may start by stage 710 of receiving detection signals from multiple sensors; wherein the multiple sensors comprise a first sensor that is positioned such as to be contacted by a first hand of a user when the user holds the hand-held device and a second sensor that is positioned such as to be contacted by a second hand of the user when the user holds the hand-held device; wherein at least one sensor of the first sensor and the second sensor is a hybrid sensor that comprises an electrode, an illumination element and a light detector.

Stage 710 may be followed by stage 720 of processing, by a health monitoring module, the detections signals from at least the electrode and from the light detector such as to provide processed signals that are indicative of a state of the user.

The hand-held device 20 that executes method 700 can be any of the mentioned above hand-held devices.

For example, stage 710 can include at least one of the following:

1. Receiving detection signals from a hybrid sensor that includes an electrode that defines a light illumination aperture and a light collection aperture; wherein the illumination element is arranged to direct light towards the user through the light illumination aperture; and wherein the light detector is arranged to detect light from the user that passes through the light collection aperture.

2. Receiving detection signals from a hybrid sensor that includes an electrode that defines a light illumination aperture and multiple light collection apertures; wherein the illumination element is arranged to direct light towards the user through the light illumination aperture; and wherein at least one light detector is arranged to detect light from the user that passes through the multiple light collection apertures.

3. Receiving detection signals from a hybrid sensor that includes a light illumination aperture that is positioned between a pair of light collection apertures.

4. Receiving detection signals from a hybrid sensor that includes at least one light detector that is shielded by an apertured shield.

5. Receiving detection signals from a hybrid sensor that includes multiple illumination elements and multiple light detectors that are spaced apart from each other.

6. Receiving detection signals from a hybrid sensor that includes an electrode, a light detector and an illumination element that are proximate to each other.

7. Receiving detection signals from a third sensor that is positioned such as to be contacted by the first or second hand of the user when the user holds the hand-held device. The third sensor can be a hybrid sensor or can differ from a hybrid sensor.

8. Receiving detection signals from a third sensor that is positioned at a first side of the hand-held device while the first and second sensors are positioned at a second side of the hand-held device, the second side is opposite to the first side.

9. Receiving detection signals from a third sensor that is positioned such as to be contacted by a thumb of one of the hands of the user while the first and second sensors are positioned such as to be contacted by index fingers of the user.

10. Receiving detection signals from a fourth sensor that is positioned such as to be contacted by the hand of the user that differs from a hand of the user that contacts the third sensor.

11. Receiving detection signals from a hybrid sensor that includes an electrode that includes a conductive portion and at least one additional portion. The additional portion may be insulating or partially conductive. The additional portion may be thicker (for example - at least three times thicker) than conductive portion.

For example, stage 720 can include at least one of the following:

1. Performing, by the health monitoring module, a common noise rejection algorithm on detection signals received from electrodes of multiple sensors out of the first, second and third sensors.

2. Performing, by the health monitoring module, the common noise rejection algorithm on detection signals received from electrodes of the first, second and third sensors.

3. Processing, by the health monitoring module, detection signals from the light detector to provide an indication about a blood oxygen saturation level of the user.

4. Processing, by the health monitoring module, detection signals from the electrode to provide an indication about an electrical activity of a heart of the user.

5. Processing, by the health monitoring module, detection signals from the light detector to provide an indication about an electrical activity of a heart of the user.

6. Correlating, by the health monitoring module, between the detection signals of the light detector and of the electrode to provide an indication about an electrical activity of a heart of the user.

7. Processing, by the health monitoring module, the detection signals of the light detector to define a processing window for processing the detection signals of the electrode.

8. Processing, by the health monitoring module, the detection signals of the light detector to detect a QRS complex; defining an expected timing of a detection of a QRS complex in the detection signals of the electrode; and searching for the QRS complex in detection signals of the electrode that are detected in proximity to the expected timing of detection.

9. Method 700 can include stage 730 of controlling the operation of the electrode and of the illumination elements. Stage 730 may include activating the illumination element and the light detector of the hybrid sensor while collecting detection signals from the electrode. Stage 730 may include ignoring detection signals from the electrode while measuring a blood oxygen saturation of the user.

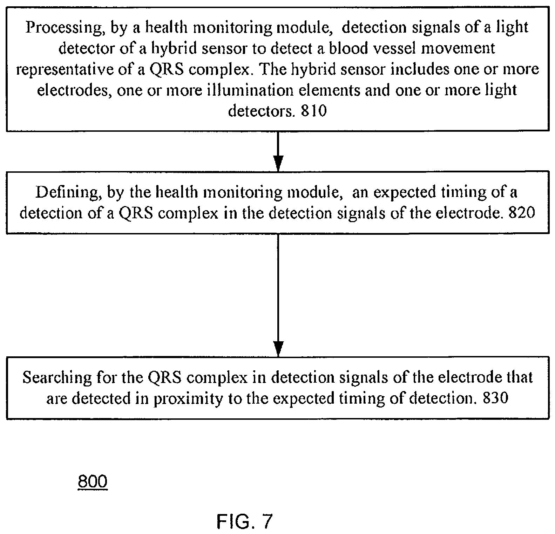

Figure 7 illustrates method 800.

Method 800 may start by stage 810 of processing, by a health monitoring module, detection signals of a light detector of a hybrid sensor to detect a blood vessel movement representative of a QRS complex. The hybrid sensor includes one or more electrodes, one or more illumination elements and one or more light detectors.

Stage 810 is followed by stage 820 of defining, by the health monitoring module, an expected timing of a detection of a QRS complex in the detection signals of the electrode.

Stage 820 may be followed by stage 830 of searching for the QRS complex in detection signals of the electrode that are detected in proximity to the expected timing of detection. A non-limiting example of an execution of method 800 can be found in figure 6.

There is provided a method for monitoring heart related parameters. The method may include detecting QRS complexes on ECG signal, detecting pulsing activities on PPG signals, phase matching and at lease zero optimization stages out of (a) optimal estimation of HR for Bradycardia and Tachycardia detection, and (b) Optimal estimation of HRV for AFIB detection.

The Detection of QRS complexes on ECG signal may include receiving detection signals from one or more electrodes and then differentiating the detection signals in order to get QRS complex slope data.

The following filter can be used to approximate that derivative ($X_n$, $X_{n+1}$ and $X_{n+2}$ are samples of the detection signal)

$$y_n = -x_{n-2} - 2*x_{n-1} + 2*x_{n+1} + x_{n+2}$$

The resultant signal (Yn) is compared to a set of adaptive thresholds to make the final decision (together with the noise detection results).

The detection of pulsing activity on PPG signal may include preprocessing and peak detection.

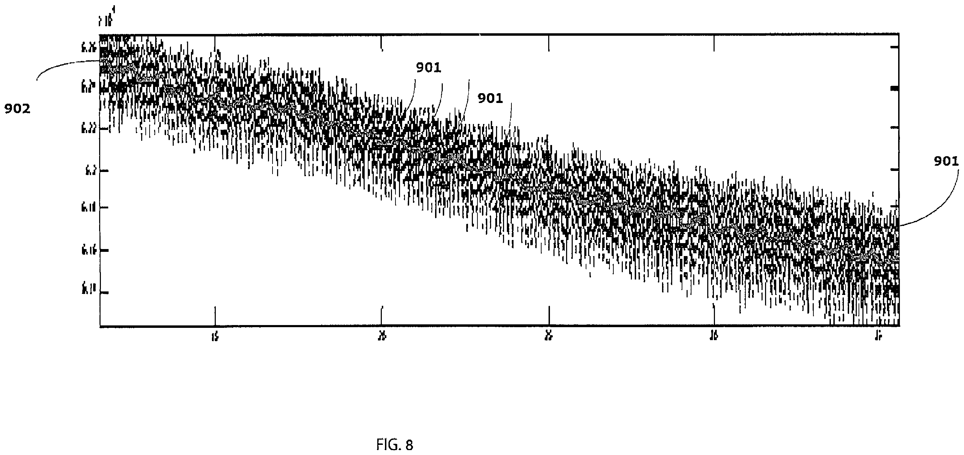

The preprocessing may include filtering the PPG signal (for example using a finite impulse response filter with 128 taps between 0.5 and 4 Hz. The outcome of this filtering is a filtered signal. In figure 8 the filtered signal is represented by line 902 and the PPG signal is represented by curves 901. The filtered signal 902 shows a pulsing activity where each pulse corresponds to a single heartbeat.

The peak detection includes detecting peaks which correspond to each heartbeat. These peaks are identified by testing whether within each N samples the maximum value appears on sample N/2. N is adjusted so small maxima are not found. The dots 903 of filtered signal 902 represent some of these peaks. The number of those peaks within a given minute will give the HR in beat per minute (BPM) units.

Two sources of information (PPG pulse timing and QRS pulse timing) both report on the temporal location of the heart contraction and therefore they can be combined and therefore improve the QRS detection. Two problems have to be overcome in order to combine the sources of information: (A) False positive and miss detection of complexes in both the signals and (B) the relative temporal shift between the two sources of information.

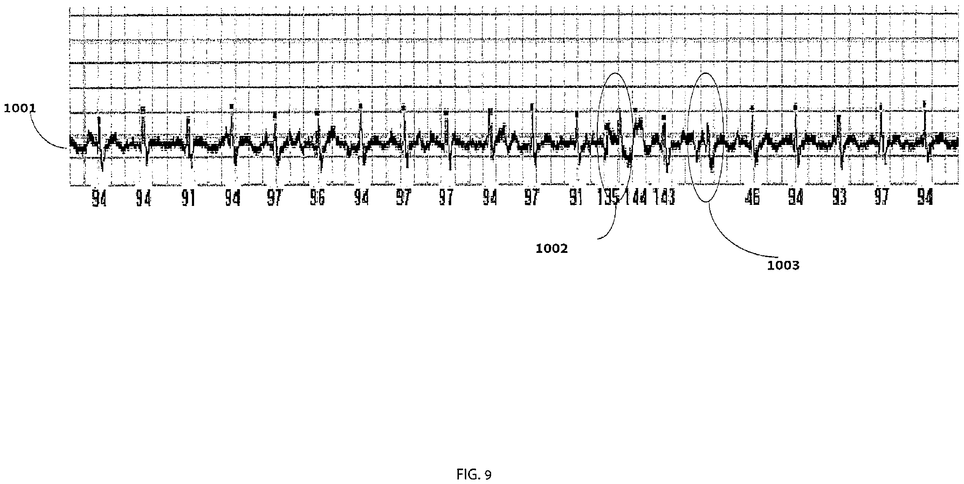

Figure 9 illustrates an ECG signal 1001. A first ellipse 1002 shows a false detection of a QRS complex. A second ellipse 1003 shows a missed QRS complex.

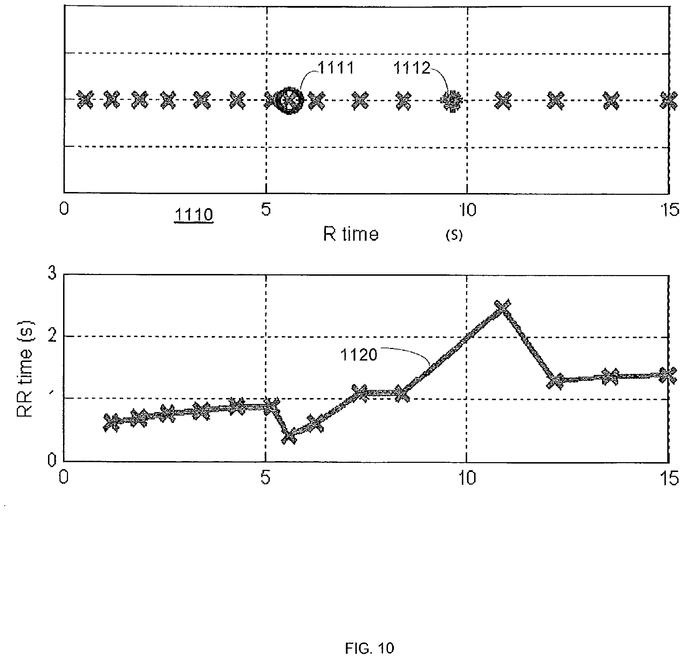

In figure 10 an ECG trace is shown along with beat by beat heart rate (numbers on the bottom of the figure) which are derived by taking the difference in QRS timing. In cases where a QRS is missed and falsely detected the HR which should be around 90 BPM would shift to 144 or 46. The same is true for the PPG signal where complexes might be falsely detected or missed. In order to match between the two sets of detections these false detections and missed complexes should be removed.

False and negative detections may be are removed by fitting a polynomial model (for example- of a third order3) to the RR sequence.

The RR sequence is generated by taking the difference in time between two consecutive QRS complexes. A missed QRS complex within the sequence will create a large entry whereas a false detection will create a rather small entry into the sequence.

Figure 10 shows a sequence of detection time for QRS complexes. The top graph 1100 shows the timing of each QRS. The circle 1111 marks a false detection and the magenta asterisk 1112 corresponds to a false detection (the complex was not found).

The bottom graph 1120 shows the RR sequence. It is evident that the false detection (1111) leads to a momentary decrease in RR value. The miss detected QRS complex (1112) led to a large value in the RR sequence.

Once the RR sequence is estimated the method can perform one or more iterations of:

12. Estimating a polynomial model (order 3) to the current RR sequence.
13. Calculating the estimated RR sequence based on the model - call it *Err*
14. Calculating an error term *e = RR-eRR*. In this error term find large entries (*lErr*) and small entries (*sErr*). Essentially the *lErr* terms correspond to missed QRS complexes which result in a high value of RR. The *sErr* correspond to false detections. And
15. Searching for out layers (*lErr* and *sErr*). Remove *sErr*. Store the *lErr*.

Stages 1 - 4 can be repeated until no out layers are found.

Relative temporal shift between PPG and ECG R location.

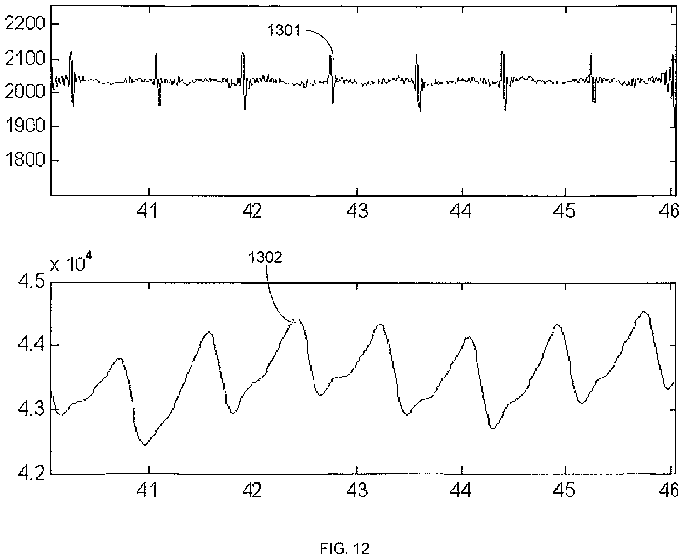

Figure 12 illustrates an example of a RR sequence 1201 and an estimated RR sequence (eRR) 1202.

The estimated phase and optimal RR interval can be derived for the remaining sequence of R (after removing *lErr* and *sErr* - see above). The optimal RR interval can be: RRopt=argmax over RR of (absolute value of (sum over RR of e by the power of (j*2*Pi*R(n)/(RR(n)))),

wherein RR ranges between minRR and maxRR.

Once the optimal RR value is found (RR opt) the angle (or phase) of each R entry can be calculated by: angle (n)=e by the power of (j*2*Pi*R(n)/(RRopt).

The optimal RR and angle is evaluated or both the QRS complexes and the PPG output. The two outputs are then compared.

Three ways can be used:

1. Calculating a goodness metric for each measurement, ECG and PPG(usually between 0 to 1) and then combining the two by a weighted sum of the two outputs.
2. Statistical comparison - matching the statistics of $Angle_{QRS}$ and $Angle_{PPG}$ by using the Kullback-Leibler divergence.
3. Direct matching - testing the agreement of each entry of one sequence ($Angle_{PPG}$) with the other ($Angle_{QRS}$).

Assuming the angle (both PPG and QRS) has a normal distribution the overall agreement between $Angle_{QRS}$ and $Angle_{PPG}$ is evaluated by:

$$L_T = \prod_i L_i \left( Angle(i)_{PPG}, N(\mu_{QRS}, \sigma_{QRS}) \right)$$

Where $L$ is the likelihood function between a single sample (of $Angle_{PPG}$) in this case and the distribution of $Angle_{QRS}$.

$$L(X, N(\mu, \sigma)) = \frac{1}{\sqrt{2\pi}\sigma} e^{-\left(\frac{X-\mu}{2\sigma}\right)^2}$$ Figure 12 illustrates the ECG signal 1301, the PPG 1302, as a function of time. It is evident that every ECG QRS complex matches with a peak in the PPG signal.

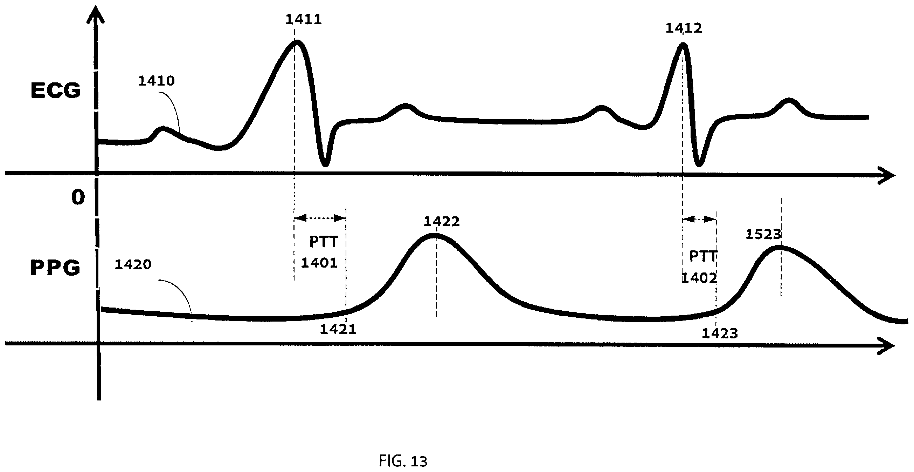

Figure 13 illustrates filtered ECG and filtered PPG signals.

Curve 1410 represents the filtered ECG signals and it includes two peaks (second points in time) 1411 and 1412. Curve 1420 represents the filtered PPG signals and it includes two peaks 1422 and 1523 and two start points (first points in time) 1421 and 1423 that represent the beginning of the pulse.

This figure shows two PTTs- a first PTT 1401 is the difference between points in time 1411 and 1421 and the second PTT is the difference between points in time 1412 and 1423.

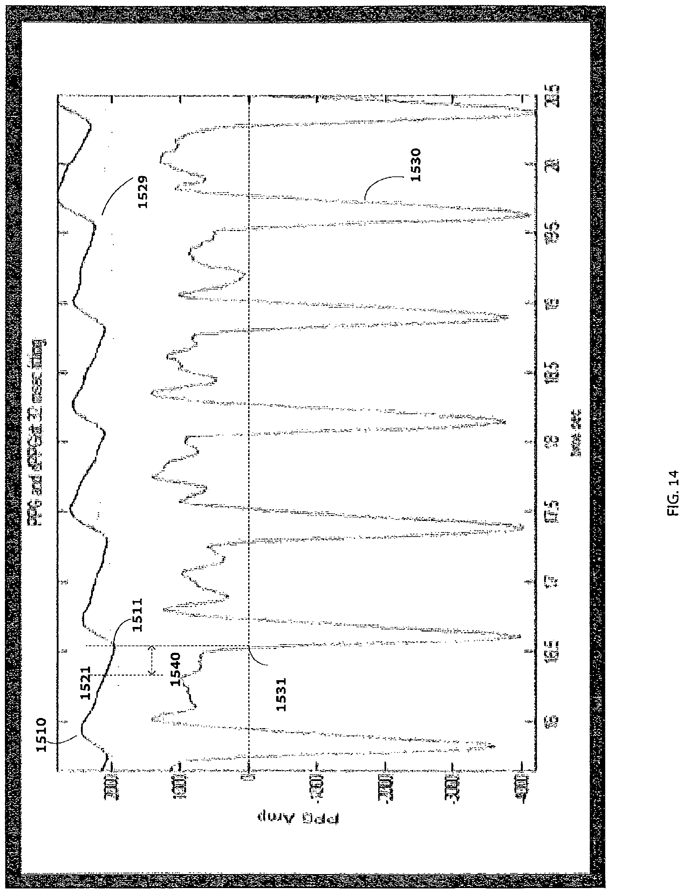

Figure 14 illustrates filtered ECG, filtered PPG signals and a derivative of the filtered PPG signals.

Curve 1520 represents the filtered ECG signals and it includes peaks (second points in time) such as peak 1521.

Curve 1510 represents the filtered PPG signals and it includes starts (foots) of pulses (first points in time) such as 1511. First point in time 1511 occurs when the value of the derivative of the filtered PPG signals equals zero (at point 1521 of curve 1530).

There are provided methods for calculating blood pressure indicators according to claim 1. A blood pressure indicator can be indicative of a blood pressure of a person including but not limited to a mean blood pressure, a diastolic blood pressure a systolic blood pressure and the like.

The value of the blood pressure indicator can be the value of the blood pressure of the person or may differ from the value of the blood pressure of the person. For example, the value of the blood pressure indicator can represent the pulse transfer time (PTT) of the person. The blood pressure can be responsive to various variables in addition to the PTT so that in some cases changes in values of blood pressure indicators may provide an indication of changes in the blood pressure - even if the exact value of the blood pressure is not known.

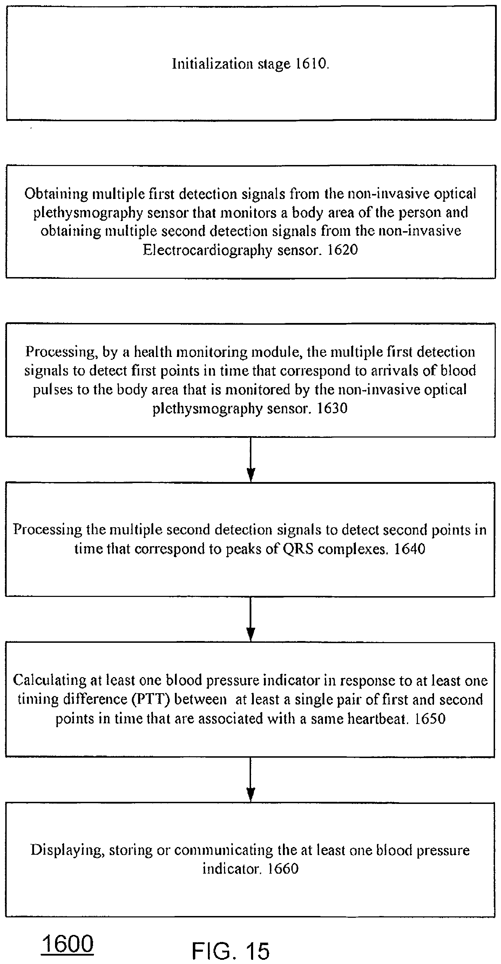

Figure 15 illustrates method 1600 for providing a blood pressure indicator according to an embodiment of the invention.

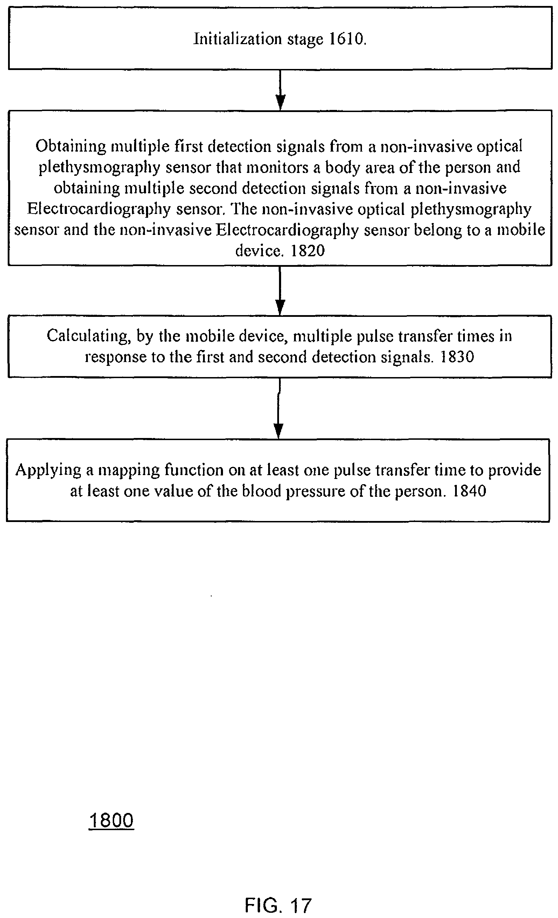

Method 1600 may start by an initialization stage 1610. During stage 1610 the relationship between the PTT and the blood pressure can be determined. Additionally such information about the relationship between the PTT and the blood pressure can be received. The information can be a mapping function or one or more correlation coefficients. Figure 17 illustrates a method 1700 for calibration during which such information can be obtained.

The initialization stage 1610 may include placing a mobile device in proximity to a person in order to monitor a body area of the person. The mobile device may include sensors such as a non-invasive optical plethysmography sensor and a non-invasive Electrocardiography sensor. The placement may include allowing a person to contact the mobile phone.

Stage 1610 may be followed by stage 1620 of obtaining multiple first detection signals from the non-invasive optical plethysmography sensor that monitors a body area of the person and obtaining multiple second detection signals from the non-invasive Electrocardiography sensor.

Stage 1620 can be executed by any of the devices illustrated above.

Stage 1620 may be followed by stages 1630 and 1640.

Stage 1630 may include processing, by a health monitoring module, the multiple first detection signals to detect first points in time that correspond to arrivals of blood pulses to the body area that is monitored by the non-invasive optical plethysmography sensor. Stage 1630 may include at least one out of: (a) low-pass filtering the multiple first detection signals to provide multiple first filtered detection signals; (b) calculating a derivative of the first filtered detection signals and detecting the first points in time in response to values of the derivative; (c) calculating the derivative of the first filtered detection signals by applying a least squares parabolic differential filter; (d) detecting first points in time be having a value that is a predetermined fraction (or within a predetermined fraction range) of the maximal value of the maximal filtered first detection signals.

Stage 1640 may include processing the multiple second detection signals to detect second points in time that correspond to peaks of QRS complexes.

Stages 1630 and 1640 may be followed by stage 1650 of calculating at least one blood pressure indicator in response to at least one timing difference (PTT) between at least a single pair of first and second points in time that are associated with a same heartbeat.

Stage 1650 may include at least one of the following stages: (a) calculating a blood pressure indicator per each PTT, (b) calculating a blood pressure indicator per multiple PTTs, (c) comparing different blood pressure indicators to provide an indication of a trend of changes in a blood pressure of the person, (c) calculating the blood pressure indicator in response to at least one correlation coefficient that correlates between one or more PTTs and the one or more PTTs. Stage 1650 may be followed by stage 1660 of displaying, storing or communicating the at least one blood pressure indicator.

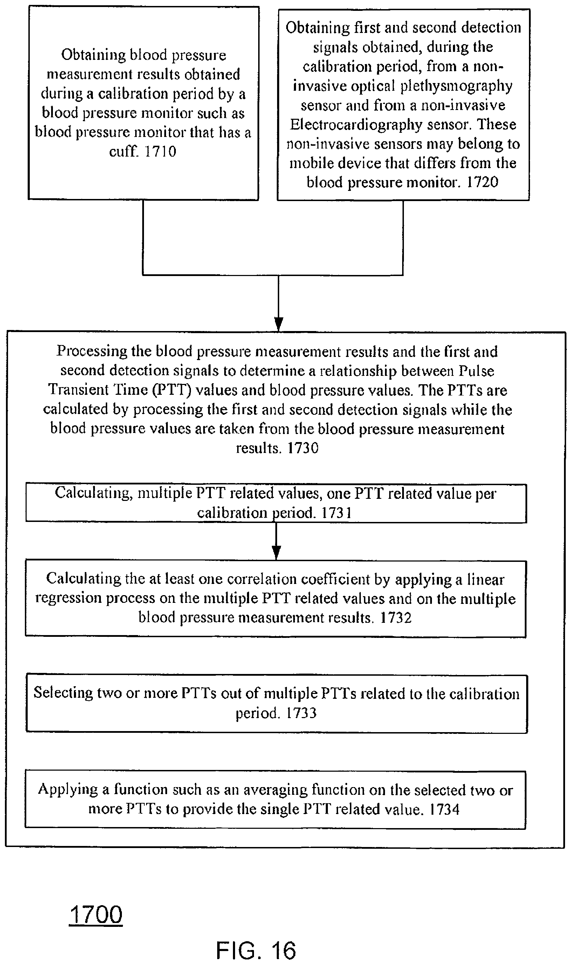

Figure 16 illustrates method 1700 according to an embodiment of the invention.

Method 1700 can be executed randomly, in a pseudo-random manner, in a periodic manner (every few hours, every few days, every few weeks...), in response to events (such as an occurrence of unacceptable measurement errors) and the like.

More frequent calibration sequences may be followed by more accurate results.

Method 1700 may start by stages 1710 and 1720. Stages 1710 and 1720 are executed during a calibration period. According to an embodiment of the invention stages 1710 and 1720 may be repeated for multiple calibration periods.

Stage 1710 includes obtaining blood pressure measurement results by a blood pressure monitor such as blood pressure monitor that has a cuff.

Stage 1720 may include obtaining first and second detection signals obtained, during the multiple calibration periods, from a non-invasive optical plethysmography sensor and from a non-invasive Electrocardiography sensor. These non-invasive sensors may belong to mobile device that differs from the blood pressure monitor.

Stages 1710 and 1720 may be followed by stage 1730 of processing the blood pressure measurement results and the first and second detection signals to determine a relationship between Pulse Transient Time (PTT) values and blood pressure values. The PTTs are calculated by processing the first and second detection signals while the blood pressure values are taken from the blood pressure measurement results.

The mapping function according to the invention equals:

Blood pressure = $-A*\ln(PTT)+B+A*\ln(L)$; wherein ln represent the logarithmic operation, A and B and correlation coefficients, and L is a length of a pressure wave path. The blood pressure can be the systolic blood pressure, the diastolic blood pressure , a mean blood pressure and the like.

Other mapping functions, not according to the invention, are:

- DBP=SBP0/3 + 2DBP0/3+$C*\ln(PTTwo/PTTw)$-((SBP0-DBP0)/3)*(PTTw0/PTTw)*(PTTw0/PTTw), wherein SBP is the systolic blood pressure, DBP is the diastolic blood pressure, SBP0 and DBP0 are SBP and DBP values obtained during a calibration period, PTTw is an average PTT measured in present time and PTTw0 is an average PTT measured in previous time.
- SBP=DBP+(SBP0-DBP0)*(PTTwo/PTTw))*(PTTwo/PTTw).
- $SBP_{PTT} = P1*PWV*\exp(P3*PWV + P2*PWV^{P4} - (BP_{PTTcal} - BP_{cal})$, wherein $BP_{PTTcal}$ is the calculated BP (from PTT) corresponding to the $BP_{cal}$ measured by the reference method (cuff) at a distinct time at the beginning of the experiment. The parameters P1-P4 were estimated by least square fitting of the function to the data of multiple persons and PWV (cm/ms) = 0.5* height(cm) / PTT.
- $SBP = P_B - (2/(\alpha*T_B)*\Delta T$, where $\Delta T$ is the change in the PTT over time, $T_B$ is the value of the PTT corresponding to the pressure $P_B$.
- $BP = aPTT + b$.
- $SBP = a1 \cdot PAT+b1 \cdot HR+c1$.
- $DBP = a2 \cdot PAT+b2 \cdot HR+c2$.
  Wherein PAT is a pulse arrival time. PAT equals PTT + aorta valve opening delay , HR is a heart rate, correlation coefficients a1, a2, b1, b2, c1 and c2 can be calculated by adaptive filtering on base of minimum least squares criterion.

It is noted that with an acute rise in blood pressure (BP), vascular tone increases - the arterial wall becomes stiffer causing the PTT to shorten. In contrast, when the BP falls there is relaxation of vascular tone and the PTT increases. In addition, arteries stiffen with age, arteriosclerosis and diabetes mellitus, also resulting in a shortening of the PTT. Thus- without a mapping function that takes such parameters into account it is hard to provide an accurate measurement of the blood pressure itself.

Stages 1710 and 1720 may be repeated multiple times, over multiple calibration periods and stage 1730 may include (a) stage 1731 of calculating, multiple PTT related values, one PTT related value per calibration period; and (b) stage 1732 of calculating the at least one correlation coefficient by applying a linear regression process on the multiple PTT related values and on the multiple blood pressure measurement results.

A PTT related value can be the PTT itself, or can be an outcome of processing one or more PTTs obtained during one or more calibration values.

For example, a single PTT related value can be calculated (during stage 1730) per a single calibration period by a process that may include: (a) selecting (stage 1733) two or more PTTs out of multiple PTTs related to the calibration period, and (b) applying (stage 1734) a function such as an averaging function on the selected two or more PTTs to provide the single PTT related value. The selecting may include clustering the PTTs values, and selecting the two or more PTT values that form a cluster that includes PTT values that are relatively close to each other.

The selecting may include ignoring PTTs if their values is outside an allowable range of timing difference values.

Figure 17 illustrates method 1800 according to an embodiment of the invention. Method 1800 may start by initialization stage 1810 that may resemble stage 1610 of method 1600.

Stage 1810 may be followed by stage 1820 of obtaining multiple first detection signals from a non-invasive optical plethysmography sensor that monitors a body area of the person and obtaining multiple second detection signals from a non-invasive Electrocardiography sensor. The non-invasive optical plethysmography sensor and the non-invasive Electrocardiography sensor belong to a mobile device.

Stage 1810 may include calculating the mapping function based upon blood pressure measurement results obtained by a blood pressure monitor that differs from the mobile device. Stage 1810 may include any of the stages of method 1700.

Stage 1820 may be followed by stage 1830 of calculating, by the mobile device, multiple pulse transfer times in response to the first and second detection signals.

Stage 1830 may be followed by stage 1840 of applying a mapping function on at least one pulse transfer time to provide at least one value of the blood pressure of the person.

Stage 1820 may include processing the multiple first detection signals to detect first points in time that correspond to arrivals of blood pulses to the body area that is monitored by the non-invasive optical plethysmography sensor; processing the multiple second detection signals to detect second points in time that correspond to peaks of QRS complexes; and calculating at least one timing difference between at least a single pair of first and second points in time that are associated with a same heartbeat.

Stage 1820 may include processing the first and second detection signals to find points in time that differ from the first points and the second points in time.

There is provided a non-transitory computer readable medium according to claim 13 that can store instructions for executing any of the mentioned above methods or any combination of any two or more stages of any of the mentioned above methods.

The disclosure also refers to the implementation of the above aspects in a computer program for running on a computer system, at least including code portions for performing steps of a method according to the invention when run on a programmable apparatus, such as a computer system or enabling a programmable apparatus to perform functions of a device according to the invention.

A computer program is a list of instructions such as a particular application program and/or an operating system. The computer program may for instance include one or more of: a subroutine, a function, a procedure, an object method, an object implementation, an executable application, an applet, a servlet, a source code, an object code, a shared library/dynamic load library and/or other sequence of instructions designed for execution on a computer system.

The computer program may be stored internally on a non-transitory computer readable medium. All or some of the computer program may be provided on computer readable media permanently, removably or remotely coupled to an information processing system. The computer readable media may include, for example and without limitation, any number of the following: magnetic storage media including disk and tape storage media; optical storage media such as compact disk media (e.g., CD-ROM, CD-R, etc.) and digital video disk storage media; nonvolatile memory storage media including semiconductor-based memory units such as FLASH memory, EEPROM, EPROM, ROM; ferromagnetic digital memories; MRAM; volatile storage media including registers, buffers or caches, main memory, RAM, etc.

A computer process typically includes an executing (running) program or portion of a program, current program values and state information, and the resources used by the operating system to manage the execution of the process. An operating system (OS) is the software that manages the sharing of the resources of a computer and provides programmers with an interface used to access those resources. An operating system processes system data and user input, and responds by allocating and managing tasks and internal system resources as a service to users and programs of the system.

The computer system may for instance include at least one processing unit, associated memory and a number of input/output (I/O) devices. When executing the computer program, the computer system processes information according to the computer program and produces resultant output information via I/O devices.

In the foregoing specification, the invention has been described with reference to specific examples of embodiments of the invention. It will, however, be evident that various modifications and changes may be made therein without departing from the scope of the invention as set forth in the appended claims.

Moreover, the terms "front," "back," "top," "bottom," "over," "under" and the like in the description and in the claims, if any, are used for descriptive purposes and not necessarily for describing permanent relative positions. It is understood that the terms so used are interchangeable under appropriate circumstances such that the embodiments of the invention described herein are, for example, capable of operation in other orientations than those illustrated or otherwise described herein.

The connections as discussed herein may be any type of connection suitable to transfer signals from or to the respective nodes, units or devices, for example via intermediate devices. Accordingly, unless implied or stated otherwise, the connections may for example be direct connections or indirect connections. The connections may be illustrated or described in reference to being a single connection, a plurality of connections, unidirectional connections, or bidirectional connections. However, different embodiments may vary the implementation of the connections. For example, separate unidirectional connections may be used rather than bidirectional connections and vice versa. Also, plurality of connections may be replaced with a single connection that transfers multiple signals serially or in a time multiplexed manner. Likewise, single connections carrying multiple signals may be separated out into various different connections carrying subsets of these signals. Therefore, many options exist for transferring signals.

Although specific conductivity types or polarity of potentials have been described in the examples, it will be appreciated that conductivity types and polarities of potentials may be reversed.

Each signal described herein may be designed as positive or negative logic. In the case of a negative logic signal, the signal is active low where the logically true state corresponds to a logic level zero. In the case of a positive logic signal, the signal is active high where the logically true state corresponds to a logic level one. Note that any of the signals described herein may be designed as either negative or positive logic signals. Therefore, in alternate embodiments, those signals described as positive logic signals may be implemented as negative logic signals, and those signals described as negative logic signals may be implemented as positive logic signals.

Furthermore, the terms "assert" or "set" and "negate" (or "deassert" or "clear") are used herein when referring to the rendering of a signal, status bit, or similar apparatus into its logically true or logically false state, respectively. If the logically true state is a logic level one, the logically false state is a logic level zero. And if the logically true state is a logic level zero, the logically false state is a logic level one.

Those skilled in the art will recognize that the boundaries between logic blocks are merely illustrative and that alternative embodiments may merge logic blocks or circuit elements or impose an alternate decomposition of functionality upon various logic blocks or circuit elements. Thus, it is to be understood that the architectures depicted herein are merely exemplary, and that in fact many other architectures may be implemented which achieve the same functionality.

Any arrangement of components to achieve the same functionality is effectively "associated" such that the desired functionality is achieved. Hence, any two components herein combined to achieve a particular functionality may be seen as "associated with" each other such that the desired functionality is achieved, irrespective of architectures or intermedial components. Likewise, any two components so associated can also be viewed as being "operably connected," or "operably coupled," to each other to achieve the desired functionality.

Furthermore, those skilled in the art will recognize that boundaries between the above described operations merely illustrative. The multiple operations may be combined into a single operation, a single operation may be distributed in additional operations and operations may be executed at least partially overlapping in time. Moreover, alternative embodiments may include multiple instances of a particular operation, and the order of operations may be altered in various other embodiments.

Also for example, in one embodiment, the illustrated examples may be implemented as circuitry located on a single integrated circuit or within a same device. Alternatively, the examples may be implemented as any number of separate integrated circuits or separate devices interconnected with each other in a suitable manner.

Also for example, the examples, or portions thereof, may implemented as soft or code representations of physical circuitry or of logical representations convertible into physical circuitry, such as in a hardware description language of any appropriate type.

Also, the invention is not limited to physical devices or units implemented in non-programmable hardware but can also be applied in programmable devices or units able to perform the desired device functions by operating in accordance with suitable program code, such as mainframes, minicomputers, servers, workstations, personal computers, notepads, personal digital assistants, electronic games, automotive and other embedded systems, cell phones and various other wireless devices, commonly denoted in this application as 'computer systems'.

However, other modifications, variations and alternatives are also possible. The specifications and drawings are, accordingly, to be regarded in an illustrative rather than in a restrictive sense.

In the claims, any reference signs placed between parentheses shall not be construed as limiting the claim. The word 'comprising' does not exclude the presence of other elements or steps then those listed in a claim. Furthermore, the terms "a" or "an," as used herein, are defined as one or more than one. Also, the use of introductory phrases such as "at least one" and "one or more" in the claims should not be construed to imply that the introduction of another claim element by the indefinite articles "a" or "an" limits any particular claim containing such introduced claim element to inventions containing only one such element, even when the same claim includes the introductory phrases "one or more" or "at least one" and indefinite articles such as "a" or "an." The same holds true for the use of definite articles. Unless stated otherwise, terms such as "first" and "second" are used to arbitrarily distinguish between the elements such terms describe. Thus, these terms are not necessarily intended to indicate temporal or other prioritization of such elements The mere fact that certain measures are recited in mutually different claims does not indicate that a combination of these measures cannot be used to advantage.

**Claims**

1.  A method (1600, 1700, 1800) for providing a blood pressure indicator of a person, the method comprises:

    obtaining (1620, 1720, 1820) multiple first detection signals from a non-invasive optical plethysmography sensor that monitors a body area of the person;
    obtaining multiple second detection signals from a non-invasive Electrocardiography sensor;
    processing (1630), by a health monitoring module, the multiple first detection signals to detect first points in time that correspond to arrivals of blood pulses to the body area that is monitored by the non-invasive optical plethysmography sensor;
    processing (1640) the multiple second detection signals to detect second points in time that correspond to peaks of QRS complexes;
    calculating (1650) at least one blood pressure indicator in response to at least one timing difference (pulse transient time) between at least a single pair of first and second points in time that are associated with a same heartbeat;
    **characterized by**
    obtaining (1710) blood pressure measurement results by a blood pressure monitor that differs from the non-invasive optical plethysmography sensor and from the non-invasive Electrocardiography sensor and calculating (1730) a mapping function that represents a relationship between pulse transient time values and blood pressure values,
    wherein the mapping function equals -A*ln(PTT)+B+A*ln(L); wherein ln represent the logarithmic operation, A and B are correlation coefficients, and L is a length of a pressure wave path.

2.  The method according to claim 1, wherein the non-invasive Electrocardiography sensor comprises an electrode, the non-invasive optical plethysmography sensor comprises an illumination element (210, 350) and a light detector (200(1)-200(N), 340, 360); and wherein the electrode (120, 310), the illumination element (210, 350) and the light detector (200(1)-200(N), 340, 360) form a hybrid sensor (70); wherein the electrode (120, 310) defines a light illumination aperture (110(1)-110(K), 313) and a light collection aperture (100(1)-100(N), 312, 314); wherein the illumination element (210, 350) is arranged to direct light towards the user through the light illumination aperture (110(1)-110(K), 313); and wherein the light detector (200(1)-200(N), 340, 360) is arranged to detect light from the user that passes through the light collection aperture (100(1)-100(N), 312, 314).

3.  The method (1600, 1700, 1800) according to claim 1, wherein the processing of the multiple first detection signals comprises low-pass filtering the multiple first detection signals to provide multiple first filtered detection signals.

4.  The method (1600, 1700, 1800) according to claim 3, further comprising calculating a derivative of the first filtered detection signals and detecting the first points in time in response to values of the derivative, and further comprising calculating the derivative of the first filtered detection signals by applying a least squares parabolic differential filter.

5.  The method (1600, 1700, 1800) according to claim 1, comprising calculating a blood pressure indicator that is indicative of a value of a blood pressure of the person in response to (a) at least one correlation coefficient that correlates between a timing difference between a pair of first and second points in time that are associated with the same heartbeat and a blood pressure; and (b) a value of the timing difference.

6.  The method (1600, 1700, 1800) according to claim 5, comprising calculating the at least one correlation coefficient in response to (a) blood pressure measurement results obtained by a blood pressure monitor during multiple calibration periods and in response to (b) first and second detection signals obtained, during the multiple calibration periods, from the non-invasive optical plethysmography sensor and the non-invasive Electrocardiography sensor, further comprising calculating, multiple timing difference value, one timing difference value per calibration period; and calculating the at least one correlation coefficient by applying a linear regression process on the multiple timing difference values and on the multiple blood pressure measurement results.

7. The method (1600, 1700, 1800) according to claim 6, comprising calculating a timing difference value for a calibration period by averaging at least two timing differences between at least two pairs of first and second points in time, each pair of first and second points in time are associated with a same heartbeat.

8. The method (1600, 1700, 1800) according to claim 6, comprising calculating a timing difference value for a calibration period by ignoring a timing difference between a pair of first and second points in time that are associated with a same heartbeat if the timing difference value is outside an allowable range of timing difference values.

9. The method (1600, 1700, 1800) according to claim 6, comprising calculating a timing difference value for a calibration period by ignoring a timing difference between a pair of first and second points in time that are associated with a same heartbeat if the timing difference value differs by at least a predetermined amount from values of timing differences that belong to cluster that comprises a majority of timing differences obtained during the measurement period.

10. A mobile device (20) that comprises:

> a non-invasive optical plethysmography sensor that monitors a body area of the person and is arranged to obtain multiple first detection signals;
> a non-invasive Electrocardiography sensor that is arranged to obtain multiple second detection signals;
> wherein the mobile device comprises:
> a health monitoring module (90) that is arranged to:
>
>> process the multiple first detection signals to detect first points in time that correspond to arrivals of blood pulses to the body area that is monitored by the non-invasive optical plethysmography sensor;
>> process the multiple second detection signals to detect second points in time that correspond to peaks of QRS complexes;
>> calculate at least one blood pressure indicator in response to at least one timing difference between at least a single pair of first and second points in time that are associated with a same heartbeat; and
>
> wherein the non-invasive Electrocardiography sensor comprises an electrode, the non-invasive optical plethysmography sensor comprises an illumination element (210, 350) and a light detector (200(1)-200(N), 340, 360); and wherein the electrode (120, 310), the illumination element (210, 350) and the light detector (200(1)-200(N), 340, 360) form a hybrid sensor (70); wherein the electrode (120, 310) defines a light illumination aperture (110(1)-110(K), 313) and a light collection aperture (100(1)-100(N), 312, 314); wherein the illumination element (210, 350) is arranged to direct light towards the user through the light illumination aperture -(100(1)-100(K), 313); and wherein the light detector (200(1)-200(N), 340, 360) is arranged to detect light from the user that passes through the light collection aperture (100(1)-100(N), 312, 314),
> **characterized in that** the mobile device comprises a blood pressure monitor that differs from the non-invasive optical plethysmography sensor and from the non-invasive electrocardiography sensor for obtaining blood pressure measurement results, the device being configured to calculate (1730) a mapping function that represents a relationship between pulse transient time values and blood pressure values, wherein the mapping function equals -A*ln(PTT)+B+A*ln(L); wherein In represent the logarithmic operation, A and B are correlation coefficients, and L is a length of a pressure wave path.

11. A non-transitory computer readable medium that stores instructions that cause a computerized system to:

> obtain (1620, 1720, 1820) multiple first detection signals from a non-invasive optical plethysmography sensor that monitors a body area of the person;
> obtain multiple second detection signals from a non-invasive Electrocardiography sensor;
> process the multiple first detection signals to detect first points in time that correspond to arrivals of blood pulses to the body area that is monitored by the non-invasive optical plethysmography sensor;
> process the multiple second detection signals to detect second points in time that correspond to peaks of QRS complexes;

calculate (1650) at least one blood pressure indicator in response to at least one timing difference between at least a single pair of first and second points in time that are associated with a same heartbeat;

obtain (1710) blood pressure measurement results by a blood pressure monitor that differs from the non-invasive optical plethysmography sensor and from the non-invasive electrocardiography sensor and calculate (1730) a mapping function that represents a relationship between pulse transient time values and blood pressure values; wherein the non-invasive Electrocardiography sensor comprises an electrode (120, 310), the non-invasive optical plethysmography sensor comprises an illumination element (210, 350) and a light detector (200(1)-200(N), 340, 360); and wherein the electrode (120, 310), the illumination element (210, 350) and the light detector (200(1)-200(N), 340, 360) form a hybrid sensor (70); wherein the electrode (120, 310) defines a light illumination aperture (110(1)-110(K), 313) and a light collection aperture (100(1)-100(N), 312, 314); wherein the illumination element (210, 350) is arranged to direct light towards the user through the light illumination aperture (110(1)-110(K), 313); and wherein the light detector (200(1)-200(N), 340, 360) is arranged to detect light from the user that passes through the light collection aperture (100(1)-100(N), 312, 314), and wherein the mapping function equals -A*ln(PTT)+B+A*ln(L); wherein ln represent the logarithmic operation, A and B are correlation coefficients, and L is a length of a pressure wave path.

**Patentansprüche**

1. Verfahren (1600, 1700, 1800) zur Bereitstellung eines Blutdruckindikators einer Person, wobei das Verfahren umfasst:

Erhalt (1620, 1720, 1820) mehrerer erster Detektionssignale von einem nichtinvasiven optischen Plethysmografie-Sensor, welcher einen Körperbereich der Person überwacht;
Erhalt mehrerer zweiter Detektionssignale von einem nichtinvasiven Elektrokardiografie-Sensor;
Verarbeiten (1630), durch ein Gesundheitsüberwachungsmodul, der mehreren ersten Detektionssignale, um erste Zeitpunkte zu detektieren, die dem Ankommen von Blutimpulsen in dem Körperbereich, der durch den nichtinvasiven optischen Plethysmografie-Sensor überwacht wird, entsprechen;
Verarbeiten (1640) der mehreren zweiten Detektionssignale, um zweite Zeitpunkte zu detektieren, die Peaks von QRS-Komplexen entsprechen;
Berechnen (1650) wenigstens eines Blutdruckindikators als Antwort auf wenigstens eine Zeitdifferenz (Pulsübergangszeit) zwischen wenigstens einem einzigen Paar erster und zweiter Zeitpunkte, die zu demselben Herzschlag gehören;
**gekennzeichnet durch**
den Erhalt (1710) von Blutdruckmessergebnissen durch einen Blutdruckmonitor, der sich von dem nichtinvasiven optischen Plethysmografie-Sensor und von dem nichtinvasiven Elektrokardiografie-Sensor unterscheidet, und das Berechnen (1730) einer Abbildungsfunktion, welche eine Beziehung zwischen Pulsübergangszeitwerten und Blutdruckwerten darstellt,
wobei die Abbildungsfunktion -A*ln(PTT)+B+A*ln(L) entspricht, wobei ln die logarithmische Operation darstellt, A und B Korrelationskoeffizienten sind, und L eine Länge eines Druckwellenwegs ist.

2. Verfahren gemäß Anspruch 1, wobei der nichtinvasive Elektrokardiografie-Sensor eine Elektrode umfasst, der nichtinvasive optische Plethysmografie-Sensor ein Beleuchtungselement (210, 350) und einen Lichtdetektor (200(1)-200(N), 340, 360) umfasst; und wobei die Elektrode (120, 310), das Beleuchtungselement (210, 350) und der Lichtdetektor (200(1)-200(N), 340, 360) einen Hybridsensor (70) bilden;
wobei die Elektrode (120, 310) eine Lichtbeleuchtungsapertur (110(1)-110(K), 313) und eine Lichtsammelapertur (100(1)-100(N), 312, 314) definiert; wobei das Beleuchtungselement (210, 350) so angeordnet ist, dass es Licht in Richtung des Anwenders durch die Lichtbeleuchtungsapertur (110(1)-110(K), 313) lenkt; und wobei der Lichtdetektor (200(1)-200(N), 340, 360) so angeordnet ist, dass er Licht von dem Anwender, welches durch die Lichtsammelapertur (100(1)-100(N), 312, 314) hindurchtritt, detektiert.

3. Verfahren (1600, 1700, 1800) gemäß Anspruch 1, wobei die Verarbeitung der mehreren ersten Detektionssignale Tiefpassfiltern der mehreren ersten Detektionssignale umfasst, um mehrere erste gefilterte Detektionssignale bereitzustellen.

4. Verfahren (1600, 1700, 1800) gemäß Anspruch 3, ferner umfassend das Berechnen einer Ableitung der ersten gefilterten Detektionssignale und Detektieren der ersten Zeitpunkte als Antwort auf Werte der Ableitung, und ferner umfassend das Berechnen der Ableitung der ersten gefilterten Detektionssignale durch Anwenden eines parabolischen Least-Squares-Differentialfilters.

5. Verfahren (1600, 1700, 1800) gemäß Anspruch 1, umfassend das Berechnen eines Blutdruckindikators, der indikativ ist für einen Blutdruckwert der Person als Antwort auf (a) wenigstens einen Korrelationskoeffizienten, der zwischen einer Zeitdifferenz zwischen einem Paar erster und zweiter Zeitpunkte, die zu demselben Herzschlag gehören, und einem Blutdruck korreliert, und (b) einen Wert der Zeitdifferenz.

6. Verfahren (1600, 1700, 1800) gemäß Anspruch 5, umfassend das Berechnen des wenigstens einen Korrelationskoeffizienten als Antwort auf (a) Blutdruckmessergebnisse, die durch einen Blutdruckmonitor während mehrerer Kalibrierungszeiträume erlangt wurden, und als Antwort auf (b) erste und zweite Detektionssignale, die im Verlauf der mehreren Kalibrierungszeiträume von dem nichtinvasiven optischen Plethysmografie-Sensor und dem nichtinvasiven Elektrokardiografie-Sensor erlangt wurden, ferner umfassend das Berechnen mehrerer Zeitdifferenzwerte, eines Zeitdifferenzwertes pro Kalibrierungszeitraum; und das Berechnen des wenigstens einen Korrelationskoeffizienten durch Anwenden eines linearen Regressionsverfahrens auf die mehreren Zeitdifferenzwerte und auf die mehreren Blutdruckmessergebnisse.

7. Verfahren (1600, 1700, 1800) gemäß Anspruch 6, umfassend das Berechnen eines Zeitdifferenzwertes für einen Kalibrierungszeitraum durch Mitteln wenigstens zweier Zeitdifferenzen zwischen wenigstens zwei Paaren erster und zweiter Zeitpunkte, wobei jedes Paar erster und zweiter Zeitpunkte zu demselben Herzschlag gehören.

8. Verfahren (1600, 1700, 1800) gemäß Anspruch 6, umfassend das Berechnen eines Zeitdifferenzwertes für einen Kalibrierungszeitraum unter Ignorieren einer Zeitdifferenz zwischen einem Paar erster und zweiter Zeitpunkte, die zu demselben Herzschlag gehören, wenn der Zeitdifferenzwert außerhalb eines zulässigen Bereichs von Zeitdifferenzwerten liegt.

9. Verfahren (1600, 1700, 1800) gemäß Anspruch 6, umfassend das Berechnen eines Zeitdifferenzwertes für einen Kalibrierungszeitraum unter Ignorieren einer Zeitdifferenz zwischen einem Paar erster und zweiter Zeitpunkte, die zu demselben Herzschlag gehören, wenn sich der Zeitdifferenzwert in wenigstens einem vorbestimmten Betrag von Zeitdifferenzwerten unterscheidet, die zu einem Cluster gehören, der einen Großteil der Zeitdifferenzen, die während des Messzeitraums erlangt werden, umfasst.

10. Mobile Vorrichtung (20) umfassend:

einen nichtinvasiven optischen Plethysmografie-Sensor, welcher einen Körperbereich einer Person überwacht und so angeordnet ist, dass er mehrere erste Detektionssignale erlangt;
einen nichtinvasiven Elektrokardiografie-Sensor, der so angeordnet ist, dass er mehrere zweite Detektionssignale erlangt;
wobei die mobile Vorrichtung umfasst:
ein Gesundheitsüberwachungsmodul (90), das so angeordnet ist, dass es:

die mehreren ersten Detektionssignale verarbeitet, um erste Zeitpunkte zu detektieren, die dem Ankommen von Blutimpulsen in dem Körperbereich, der durch den nichtinvasiven optischen Plethysmografie-Sensor überwacht wird, entsprechen;
die mehreren zweiten Detektionssignale verarbeitet, um zweite Zeitpunkte zu detektieren, die Peaks von QRS-Komplexen entsprechen;
wenigstens einen Blutdruckindikator als Antwort auf wenigstens eine Zeitdifferenz zwischen wenigstens einem einzigen Paar erster und zweiter Zeitpunkte, die zu demselben Herzschlag gehören, berechnet; und wobei der nichtinvasive Elektrokardiografie-Sensor eine Elektrode umfasst, der nichtinvasive optische Plethysmografie-Sensor ein Beleuchtungselement (210, 350) und einen Lichtdetektor (200(1)-200(N), 340, 360) umfasst; und wobei die Elektrode (120, 310), das Beleuchtungselement (210, 350) und der Lichtdetektor (200(1)-200(N), 340, 360) einen Hybridsensor (70) bilden; wobei die Elektrode (120, 310) eine Lichtbeleuchtungsapertur (110(1)-110(K), 313) und eine Lichtsammelapertur (100(1)-100(N), 312, 314) definiert; wobei das Beleuchtungselement (210, 350) so angeordnet ist, dass es Licht in Richtung des Anwenders durch die Lichtbeleuchtungsapertur (110(1)-110(K), 313) lenkt; und wobei der Lichtdetektor (200(1)-200(N), 340, 360) so angeordnet ist, dass er Licht von dem Anwender, welches durch die Lichtsammelapertur (100(1)-100(N), 312, 314) hindurchtritt, detektiert,

**dadurch gekennzeichnet, dass**

die mobile Vorrichtung einen Blutdruckmonitor, der sich von dem nichtinvasiven optischen Plethysmografie-Sensor und dem nichtinvasiven Elektrokardiografie-Sensor unterscheidet, zum Erlangen von Blutdruckmessergebnissen umfasst, wobei die Vorrichtung so konfiguriert ist, dass sie eine Abbildungsfunktion berechnet (1730), welche eine Beziehung zwischen Pulsübergangszeitwerten und Blutdruckwerten darstellt, wobei die Abbildungsfunktion -A*ln(PTT)+B+A*ln(L) entspricht, wobei ln die logarithmische Operation darstellt, A und B Korrelationskoeffizienten sind, und L eine Länge eines Druckwellenwegs ist.

**11.** Nicht-transitorisches computerlesbares Medium, welches Befehle speichert, die ein computergestütztes System dazu veranlasst:

mehrere erste Detektionssignale von einem nichtinvasiven optischen Plethysmografie-Sensor, welcher einen Körperbereich einer Person überwacht, zu erlangen (1620, 1720, 1820);
mehrere zweite Detektionssignale von einem nichtinvasiven Elektrokardiografie-Sensor zu erlangen;
die mehreren ersten Detektionssignale zu verarbeiten, um erste Zeitpunkte zu detektieren, die dem Ankommen von Blutimpulsen in dem Körperbereich, der durch den nichtinvasiven optischen Plethysmografie-Sensor überwacht wird, entsprechen;
die mehreren zweiten Detektionssignale zu verarbeiten, um zweite Zeitpunkte zu detektieren, die Peaks von QRS-Komplexen entsprechen;
wenigstens einen Blutdruckindikator als Antwort auf wenigstens eine Zeitdifferenz zwischen wenigstens einem einzigen Paar erster und zweiter Zeitpunkte, die zu demselben Herzschlag gehören, zu berechnen (1650);
Blutdruckmessergebnisse durch einen Blutdruckmonitor, der sich von dem nichtinvasiven optischen Plethysmografie-Sensor und dem nichtinvasiven Elektrokardiografie-Sensor unterscheidet, zu erlangen (1710) und eine Abbildungsfunktion zu berechnen (1730), welche eine Beziehung zwischen Pulsübergangszeitwerten und Blutdruckwerten darstellt;
wobei der nichtinvasive Elektrokardiografie-Sensor eine Elektrode (120, 310) umfasst, der nichtinvasive optische Plethysmografie-Sensor ein Beleuchtungselement (210, 350) und einen Lichtdetektor (200(1)-200(N), 340, 360) umfasst; und wobei die Elektrode (120, 310), das Beleuchtungselement (210, 350) und der Lichtdetektor (200(1)-200(N), 340, 360) einen Hybridsensor (70) bilden; wobei die Elektrode (120, 310) eine Lichtbeleuchtungsapertur (110(1)-110(K), 313) und eine Lichtsammelapertur (100(1)-100(N), 312, 314) definiert; wobei das Beleuchtungselement (210, 350) so angeordnet ist, dass es Licht in Richtung des Anwenders durch die Lichtbeleuchtungsapertur (110(1)-110(K), 313) lenkt; und wobei der Lichtdetektor (200(1)-200(N), 340, 360) so angeordnet ist, dass er Licht von dem Anwender, welches durch die Lichtsammelapertur (100(1)-100(N), 312, 314) hindurchtritt, detektiert; und wobei die Abbildungsfunktion -A*ln(PTT)+B+A*ln(L) entspricht, wobei ln die logarithmische Operation darstellt, A und B Korrelationskoeffizienten sind, und L eine Länge eines Druckwellenwegs ist.

## Revendications

**1.** Procédé (1600, 1700, 1800) de fourniture d'un indicateur de pression sanguine d'une personne, le procédé comprend :

l'obtention (1620, 1720, 1820) de multiples premiers signaux de détection à partir d'un capteur de pléthysmographie optique non invasif qui surveille une zone corporelle de la personne ;
l'obtention de multiples deuxièmes signaux de détection à partir d'un capteur d'électrocardiographie non invasif ;
le traitement (1630), par un module de surveillance de santé, des multiples premiers signaux de détection pour détecter des premiers points dans le temps qui correspondent à des arrivées d'impulsions sanguines dans la zone corporelle qui est surveillée par le capteur de pléthysmographie optique non invasif ;
le traitement (1640) des multiples deuxièmes signaux de détection pour détecter des deuxièmes points dans le temps qui correspondent à des pics de complexes QRS;
le calcul (1650) d'au moins un indicateur de pression sanguine en réponse à au moins une différence temporelle (temps transitoire d'impulsion) entre au moins une seule paire de premiers et deuxièmes points dans le temps qui sont associés à un même battement cardiaque ;
**caractérisé par**
l'obtention (1710) de résultats de mesure de la pression sanguine par un moniteur de pression sanguine qui diffère du capteur de pléthysmographie optique non invasif et du capteur d'électrocardiographie non invasif et le calcul (1730) d'une fonction de cartographie qui représente une relation entre des valeurs de temps transitoire d'impulsion et des valeurs de pression sanguine,

dans lequel la fonction de cartographie est égale à -A*ln(PTT)+B+A*ln(L) ; dans lequel ln représente l'opération logarithme, A et B sont des coefficients de corrélation, et L est une longueur d'un chemin d'onde de pression.

2. Procédé selon la revendication 1, dans lequel le capteur d'électrocardiographie non invasif comprend une électrode, le capteur de pléthysmographie optique non invasif comprend un élément d'éclairage (210, 350) et un détecteur de lumière (200(1)-200(N), 340, 360) ; et dans lequel l'électrode (120, 310), l'élément d'éclairage (210, 350) et le détecteur de lumière (200(1)-200(N), 340, 360) forment un capteur hybride (70) ; dans lequel l'électrode (120, 310) définit une ouverture d'éclairage de lumière (110(1)-110(K), 313) et une ouverture de collecte de lumière (100(1)-100(N), 312, 314); dans lequel l'élément d'éclairage (210, 350) est agencé pour diriger de la lumière vers l'utilisateur à travers l'ouverture d'éclairage de lumière (110(1)-110(K), 313) ; et dans lequel le détecteur de lumière (200(1)-200(N), 340, 360) est agencé pour détecter de la lumière provenant de l'utilisateur qui passe à travers l'ouverture de collecte de lumière (100(1)-100(N), 312, 314),

3. Procédé (1600, 1700, 1800) selon la revendication 1, dans lequel le traitement des multiples premiers signaux de détection comprend une filtration passe-bas des multiples premiers signaux de détection pour fournir de multiples premiers signaux de détection filtrés.

4. Procédé (1600, 1700, 1800) selon la revendication 3, comprenant en outre le calcul d'une dérivée des premiers signaux de détection filtrés et la détection des premiers points dans le temps en réponse à des valeurs de la dérivée, et comprenant en outre le calcul de la dérivée des premiers signaux de détection filtrés par application d'un filtre différentiel parabolique des moindres carrés.

5. Procédé (1600, 1700, 1800) selon la revendication 1, comprenant le calcul d'un indicateur de pression sanguine qui indique une valeur d'une pression sanguine de la personne en réponse à (a) au moins un coefficient de corrélation qui corrèle entre une différence temporelle entre une paire de premiers et deuxièmes points dans le temps qui sont associés au même battement cardiaque et une pression sanguine ; et (b) une valeur de la différence temporelle.

6. Procédé (1600, 1700, 1800) selon la revendication 5, comprenant le calcul de l'au moins un coefficient de corrélation en réponse à (a) des résultats de mesure de la pression sanguine obtenus par un moniteur de pression sanguine pendant de multiples périodes de calibrage et en réponse à (b) des premiers et deuxièmes signaux de détection obtenus, pendant les multiples périodes de calibrage, à partir du capteur de pléthysmographie optique non invasif et du capteur d'électrocardiographie non invasif, comprenant en outre le calcul, de multiples valeurs de différence temporelle, une valeur de différence temporelle par période de calibrage ; et le calcul de l'au moins un coefficient de corrélation par application d'un processus de régression linéaire sur les multiples valeurs de différence temporelle et sur les multiples résultats de mesure de la pression sanguine.

7. Procédé (1600, 1700, 1800) selon la revendication 6, comprenant le calcul d'une valeur de différence temporelle pour une période de calibrage en faisant la moyenne d'au moins deux différences temporelles entre au moins deux paires de premiers et deuxièmes points dans le temps, chaque paire de premiers et deuxièmes points dans le temps sont associés à un même battement cardiaque.

8. Procédé (1600, 1700, 1800) selon la revendication 6, comprenant le calcul d'une valeur de différence temporelle pour une période de calibrage en ignorant une différence temporelle entre une paire de premiers et deuxièmes points dans le temps qui sont associés à un même battement cardiaque si la valeur de différence temporelle est en dehors d'une plage admissible de valeurs de différence temporelle.

9. Procédé (1600, 1700, 1800) selon la revendication 6, comprenant le calcul d'une valeur de différence temporelle pour une période de calibrage en ignorant une différence temporelle entre une paire de premiers et deuxièmes points dans le temps qui sont associés à un même battement cardiaque si la valeur de différence temporelle diffère d'au moins une quantité prédéterminée de valeurs de différences temporelles qui font partie du groupement qui comprend une majorité de différences temporelles obtenues pendant la période de mesure.

10. Dispositif mobile (20) qui comprend :

un capteur de pléthysmographie optique non invasif qui surveille une zone corporelle de la personne et est agencé pour obtenir de multiples premiers signaux de détection ;
un capteur d'électrocardiographie non invasif qui est agencé pour obtenir de multiples deuxièmes signaux de détection ;

dans lequel le dispositif mobile comprend :
un module de surveillance de santé (90) qui est agencé pour :

traiter les multiples premiers signaux de détection pour détecter des premiers points dans le temps qui correspondent à des arrivées d'impulsions sanguines dans la zone corporelle qui est surveillée par le capteur de pléthysmographie optique non invasif ;
traiter les multiples deuxièmes signaux de détection pour détecter des deuxièmes points dans le temps qui correspondent à des pics de complexes QRS;
calculer au moins un indicateur de pression sanguine en réponse à au moins une différence temporelle entre au moins une seule paire de premiers et deuxièmes points dans le temps qui sont associés à un même battement cardiaque ; et

dans lequel le capteur d'électrocardiographie non invasif comprend une électrode, le capteur de pléthysmographie optique non invasif comprend un élément d'éclairage (210, 350) et un détecteur de lumière (200(1)-200(N), 340, 360) ; et dans lequel l'électrode (120, 310), l'élément d'éclairage (210, 350) et le détecteur de lumière (200(1)-200(N), 340, 360) forment un capteur hybride (70); dans lequel l'électrode (120, 310) définit une ouverture d'éclairage de lumière (110(1)-110(K), 313) et une ouverture de collecte de lumière (100(1)-100(N), 312, 314); dans lequel l'élément d'éclairage (210, 350) est agencé pour diriger de la lumière vers l'utilisateur à travers l'ouverture d'éclairage de lumière (110(1)-110(K), 313); et dans lequel le détecteur de lumière (200(1)-200(N), 340, 360) est agencé pour détecter de la lumière provenant de l'utilisateur qui passe à travers l'ouverture de collecte de lumière (100(1)-100(N), 312, 314),
**caractérisé en ce que** le dispositif mobile comprend un moniteur de pression sanguine qui diffère du capteur de pléthysmographie optique non invasif et du capteur d'électrocardiographie non invasif pour l'obtention de résultats de mesure de la pression sanguine, le dispositif étant configuré pour calculer (1730) une fonction de cartographie qui représente une relation entre des valeurs de temps transitoire d'impulsion et des valeurs de pression sanguine, dans lequel la fonction de cartographie est égale à -A*ln(PTT)+B+A*ln(L) ; dans lequel ln représente l'opération logarithme, A et B sont des coefficients de corrélation, et L est une longueur d'un chemin d'onde de pression.

11.  Support lisible par ordinateur non transitoire qui stocke des instructions qui amènent un système informatisé à :

obtenir (1620, 1720, 1820) de multiples premiers signaux de détection à partir d'un capteur de pléthysmographie optique non invasif qui surveille une zone corporelle de la personne ;
obtenir de multiples deuxièmes signaux de détection à partir d'un capteur d'électrocardiographie non invasif ;
traiter les multiples premiers signaux de détection pour détecter des premiers points dans le temps qui correspondent à des arrivées d'impulsions sanguines dans la zone corporelle qui est surveillée par le capteur de pléthysmographie optique non invasif ;
traiter les multiples deuxièmes signaux de détection pour détecter des deuxièmes points dans le temps qui correspondent à des pics de complexes QRS ;
calculer (1650) au moins un indicateur de pression sanguine en réponse à au moins une différence temporelle entre au moins une seule paire de premiers et deuxièmes points dans le temps qui sont associés à un même battement cardiaque ;
obtenir (1710) des résultats de mesure de la pression sanguine par un moniteur de pression sanguine qui diffère du capteur de pléthysmographie optique non invasif et du capteur d'électrocardiographie non invasif et calculer (1730) une fonction de cartographie qui représente une relation entre des valeurs de temps transitoire d'impulsion et des valeurs de pression sanguine ;
dans lequel le capteur d'électrocardiographie non invasif comprend une électrode (120, 310), le capteur de pléthysmographie optique non invasif comprend un élément d'éclairage (210, 350) et un détecteur de lumière (200(1)-200(N), 340, 360) ; et dans lequel l'électrode (120, 310), l'élément d'éclairage (210, 350) et le détecteur de lumière (200(1)-200(N), 340, 360) forment un capteur hybride (70) ; dans lequel l'électrode (120, 310) définit une ouverture d'éclairage de lumière (110(1)-110(K), 313) et une ouverture de collecte de lumière (100(1)-100(N), 312, 314); dans lequel l'élément d'éclairage (210, 350) est agencé pour diriger de la lumière vers l'utilisateur à travers l'ouverture d'éclairage de lumière (110(1)-110(K), 313) ; et dans lequel le détecteur de lumière (200(1)-200(N), 340, 360) est agencé pour détecter de la lumière provenant de l'utilisateur qui passe à travers l'ouverture de collecte de lumière (100(1)-100(N), 312, 314), et dans lequel la fonction de cartographie est égale à -A*ln(PTT)+B+A*ln(L) ; dans lequel ln représente l'opération logarithme, A et B sont des coefficients de corrélation, et L est une longueur d'un chemin d'onde de pression.

Second hand 19

Second index finger 14

First index finger 12

First hand 18

30

40

80    90

Second thumb 15

First thumb 13

Hand held device 20

FIG. 1A

Second hand 19

Second index finger 14

First index finger 12

First hand 18

30

40

80    90

50

Second thumb 15

First thumb 13

Hand held device 20

FIG. 1B

Second hand 19

Second index finger 14

First index finger 12

First hand 18

50

80    90

40    30

Second thumb 15

First thumb 13

Hand held device 20

FIG. 1C

Second hand 19

Second index finger 14

First index finger 12

First hand 18

30

40

80

90

60

50

Second thumb 15

First thumb 13

Hand held device 20

FIG. 2A

Second hand 19

Second index finger 14

First index finger 12

First hand 18

50'

50

80

90

40

30

Second thumb 15

First thumb 13

Hand held device 20

FIG. 2B

FIG. 3A

FIG. 3B

FIG. 3C

FIG. 4A

FIG. 4B

FIG. 4C

FIG. 5A

FIG. 5B

*FIG. 5C*

Receiving detection signals from multiple sensors. The multiple sensors may include a first sensor that is positioned such as to be contacted by a first hand of a user when the user holds the hand-held device and a second sensor that is positioned such as to be contacted by a second hand of the user when the user holds the hand-held device. At least one sensor of the first sensor and the second sensor is a hybrid sensor that may include an electrode, an illumination element and a light detector. 710

Processing, by a health monitoring module, the detections signals from at least the electrode and from the light detector such as to provide processed signals that are indicative of a state of the user. 720

Controlling the operation of the electrode and of the illumination elements. 730

700

FIG. 6

Processing, by a health monitoring module, detection signals of a light detector of a hybrid sensor to detect a blood vessel movement representative of a QRS complex. The hybrid sensor includes one or more electrodes, one or more illumination elements and one or more light detectors. 810

Defining, by the health monitoring module, an expected timing of a detection of a QRS complex in the detection signals of the electrode. 820

Searching for the QRS complex in detection signals of the electrode that are detected in proximity to the expected timing of detection. 830

800

FIG. 7

FIG. 8

FIG. 9

FIG. 10

FIG. 11

FIG. 12

FIG. 13

FIG. 14

Initialization stage 1610.

Obtaining multiple first detection signals from the non-invasive optical plethysmography sensor that monitors a body area of the person and obtaining multiple second detection signals from the non-invasive Electrocardiography sensor. 1620

Processing, by a health monitoring module, the multiple first detection signals to detect first points in time that correspond to arrivals of blood pulses to the body area that is monitored by the non-invasive optical plethysmography sensor. 1630

Processing the multiple second detection signals to detect second points in time that correspond to peaks of QRS complexes. 1640

Calculating at least one blood pressure indicator in response to at least one timing difference (PTT) between at least a single pair of first and second points in time that are associated with a same heartbeat. 1650

Displaying, storing or communicating the at least one blood pressure indicator. 1660

<u>1600</u>          FIG. 15

Obtaining blood pressure measurement results obtained during a calibration period by a blood pressure monitor such as blood pressure monitor that has a cuff. 1710

Obtaining first and second detection signals obtained, during the calibration period, from a non-invasive optical plethysmography sensor and from a non-invasive Electrocardiography sensor. These non-invasive sensors may belong to mobile device that differs from the blood pressure monitor. 1720

Processing the blood pressure measurement results and the first and second detection signals to determine a relationship between Pulse Transient Time (PTT) values and blood pressure values. The PTTs are calculated by processing the first and second detection signals while the blood pressure values are taken from the blood pressure measurement results. 1730

Calculating, multiple PTT related values, one PTT related value per calibration period. 1731

Calculating the at least one correlation coefficient by applying a linear regression process on the multiple PTT related values and on the multiple blood pressure measurement results. 1732

Selecting two or more PTTs out of multiple PTTs related to the calibration period. 1733

Applying a function such as an averaging function on the selected two or more PTTs to provide the single PTT related value. 1734

<u>1700</u>

FIG. 16

Initialization stage 1610.

Obtaining multiple first detection signals from a non-invasive optical plethysmography sensor that monitors a body area of the person and obtaining multiple second detection signals from a non-invasive Electrocardiography sensor. The non-invasive optical plethysmography sensor and the non-invasive Electrocardiography sensor belong to a mobile device. 1820

Calculating, by the mobile device, multiple pulse transfer times in response to the first and second detection signals. 1830

Applying a mapping function on at least one pulse transfer time to provide at least one value of the blood pressure of the person. 1840

1800

FIG. 17

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 2010222652 A1